# EUROPEAN PATENT SPECIFICATION

(11) **EP 3 676 370 B1**
(45) Date of publication and mention of the grant of the patent: **20.12.2023**
(21) Application number: 18850707.3
(22) Date of filing: 29.08.2018
(51) Int. Cl.: C12N 1/20, C12N 15/62, C12N 15/74

(54) **COMPOSITIONS AND METHODS USING METHANOTROPHIC S-LAYER PROTEINS FOR EXPRESSION OF HETEROLOGOUS PROTEINS**
ZUSAMMENSETZUNGEN UND VERFAHREN MIT METHANOTROPHEN S-SCHICHT-PROTEINEN ZUR EXPRESSION HETEROLOGER PROTEINE
COMPOSITIONS ET PROCÉDÉS UTILISANT DES PROTÉINES DE COUCHE S MÉTHANOTROPHE POUR L'EXPRESSION DE PROTÉINES HÉTÉROLOGUES

(30) Priority: 29.08.2017 US 201762551502 P; 29.08.2017 US 201762551590 P
(43) Date of publication of application: 08.07.2020
(73) Proprietor: San Diego State University (SDSU) Foundation, DBA, San Diego, California 92182-1998 (US)
(72) Inventor: KALYUZHNAYA, Marina, San Diego California 92182 (US); DEMIDENKO, Oleksandr, San Diego California 92182 (US); COLLINS, David, San Diego California 92182 (US)
(74) Representative: Pinnarò, Chiara
(86) International application number: PCT/US2018/048576
(87) International publication number: WO 2019/046446

(56) References cited:
- EP-A1- 3 106 521
- WO-A2-95/19371
- WO-A2-2017/132627
- US-A1- 2004 175 705
- US-A1- 2011 189 743
- US-A1- 2015 366 992
- US-A1- 2016 237 442
- TIMMERS PEER H. A. ET AL: "Reverse Methanogenesis and Respiration in Methanotrophic Archaea", ARCHAEA, vol. 2017, 1 January 2017 (2017-01-01), pages 1-22, XP055781931, CA ISSN: 1472-3646, DOI: 10.1155/2017/1654237 Retrieved from the Internet: URL:http://downloads.hindawi.com/journals/ archaea/2017/1654237.xml>
- STRONG P J ET AL: "A methanotroph-based biorefinery: Potential scenarios for generating multiple products from a single fermentation", BIORESOURCE TECHNOLOGY, ELSEVIER, AMSTERDAM, NL, vol. 215, 22 April 2016 (2016-04-22), pages 314-323, XP029597390, ISSN: 0960-8524, DOI: 10.1016/J.BIORTECH.2016.04.099
- SHCHUKIN V N ET AL: "Primary characterization of dominant cell surface proteins of halotolerant methanotroph20Z", MICROBIOLOGY, NAUKA/INTERPERIODICA, MO, vol. 80, no. 5, 8 October 2011 (2011-10-08), pages 608-618, XP019962139, ISSN: 1608-3237, DOI: 10.1134/S0026261711050122
- V\LLENKLE C ET AL: "Construction of a functional S-layer fusion protein comprising an immunoglobulin G-binding domain for development of specific adsorbents for extracorporeal blood purification", APPLIED AND ENVIRONMENTAL MICROBIOLOGY, AMERICAN SOCIETY FOR MICROBIOLOGY, US, vol. 70, no. 3, 1 March 2004 (2004-03-01), pages 1514-1521, XP003011043, ISSN: 0099-2240, DOI: 10.1128/AEM.70.3.1514-1521.2004
- SLEYTR UWE B ET AL: "S-Layers as a basic building block in a molecular construction kit : Molecular construction kit based on S-layers", THE FEBS JOURNAL, vol. 274, no. 2, 20 December 2006 (2006-12-20), pages 323-334, XP055781933, GB ISSN: 1742-464X, DOI: 10.1111/j.1742-4658.2006.05606.x
- SCHUSTER B. ET AL: "S-Layer Proteins as Key Components of a Versatile Molecular Construction Kit for Biomedical Nanotechnology", MINI REVIEWS IN MEDICINAL CHEMISTRY, vol. 6, no. 8, 1 August 2006 (2006-08-01), pages 909-920, XP055781934, NL ISSN: 1389-5575, DOI: 10.2174/138955706777935026 Retrieved from the Internet: URL:http://homes.nano.aau.dk/fp/self-assem bling/pdf-material/protein-drugdelivery.pd f>
- ALEXANDER S RESHETNIKOV ET AL: "Diversity and phylogeny of the ectoine biosynthesis genes in aerobic, moderately halophilic methylotrophic bacteria", EXTREMOPHILES ; LIFE UNDER EXTREME CONDITIONS, SPRINGER-VERLAG, TO, vol. 15, no. 6, 5 October 2011 (2011-10-05), pages 653-663, XP019965843, ISSN: 1433-4909, DOI: 10.1007/S00792-011-0396-X
- KUNTE et al.: "Industrial production of the cell protectant ectoine: protection mechanisms, processes, and products", Current Biotechnology, vol. 3, no. 1, 31 December 2014 (2014-12-31), pages 10-25, XP002773385, DOI: doi:10.2174/22115501113026660037
- PASTOR et al.: "Ectoines in cell stress protection: uses and biotechnological production", Biotechnology Advances, vol. 28, no. 6, 1 July 2010 (2010-07-01), pages 782-801, XP027331818,
- ORATA FABINI D. ET AL: "Phylogenomic Analysis of the Gammaproteobacterial Methanotrophs (Order Methylococcales) Calls for the Reclassification of Members at the Genus and Species Levels", FRONTIERS IN MICROBIOLOGY, vol. 9, 19 December 2018 (2018-12-19), XP55950866, DOI: 10.3389/fmicb.2018.03162

## Description

### TECHNICAL FIELD

This invention generally relates to microbiology and bioengineering. Provided is a method for making a chimeric polypeptide comprising the major S-laver polypeptide of Methylomicrobium alcaliphilum sp.20Z (or a self-assembling fragment thereof) and a heterologous polypeptide or peptide. The method comprises recombinantly engineering Methylomicrobium alcaliphilum sp. 20Z to recombinantly express said chimeric polypeptide and deliver it outside of the cell where it exists in the form of an assembled monomolecular layer. It is also provided a genetically engineered Methylomicrobium alcaliphilum sp. 20Z comprising an expression cassette which recombinantly expresses said chimeric protein and an isolated assembled monomolecular layer comprising it.

### BACKGROUND

Bacterial cell surface layers are regular para-crystalline structures that cover the entire surface of a cell and consist of a single layer of identical proteins or glycoproteins. These glycoproteins are potentially of industrial interest because they intrinsically self-assemble and re-crystallise to form porous semi-permeable membranes. These characteristics, and subsequent functionalisation of surfaces, has led to new types of ultrafiltration membranes, affinity structures, enzyme membranes, micro-carriers, biosensors, diagnostic devices, biocompatible surfaces and vaccines, as well as targeting, delivery, and encapsulation.

Methylotrophic and methanotrophic bacteria have been used as systems for the heterologous expression of proteins, see e.g., US 2010 0221813 A1 (2010). However, most attempts to improve protein expression have been focused on intracellular protein expression.

An S-layer, or surface layer, a part of a cell envelope found in almost all archaea and in many types of bacteria, consists of a monomolecular layer composed of identical proteins or glycoproteins. For many bacteria, the S-layer represents the outermost interaction zone with their respective environments, and it can have many different functions depending on the species, for example an S-layer can have a mechanical and osmotic stabilization function, can protect against bacteriophages or phagocytosis, can provide resistance against low pH, can act as a barrier against high-molecular-weight substances, can act as a molecular sieve and barrier, can have antifouling properties, be involved in biomineralization, and the like.

S-Layers are present at the surfaces of methylotrophic and methanotrophic cells such as *Methylococcus, Methylothermus,* and *Methylomicrobium* bacterial cells. For example, different *Methylomicrobium* species can synthesize S-layers with planar (*p*2*, p*4) symmetry or form cup-shaped or conical structures with hexagonal (*p*6) symmetry. S-layers are a well-recognized microbial product with very broad biotechnological applications. Numerous research activities are focused on the construction of fusion proteins (S-layer proteins with attached enzymes) for production of immobilized biocatalysts. Formation of S-layers has been observed in all tested *Methylomicrobium* species. *M. album BG8, M. alcaliphilum* 20Z and *M. buryatense* form S-layers consisting of cup-shaped subunits arranged in *p6* symmetry. Methanotrophic S-layers have been mentioned as a potential value-added product, but were not explored much due to the lack of knowledge on its genetic elements. SHCHUKIN ET AL: "Primary characterization of dominant cell surface proteins of halotolerant methanotroph Methylomicrobium alcaliphilum 20Z", MICROBIOLOGY. 2011 - focuses on cell surface-associated proteins in Methylomicrobium alcaliphilum and in particular it discloses S-layer proteins as outer membrane proteins in methanotrophs and their interest for production of useful proteins by heterologous expression at the cell surface.

The use of an aerobic methane-oxidation process for methane reduction in coal mines has been actively discussed for decades and even tested in the 1980s. The approach was very simple: different methanotrophic cultures were sprayed on coal mine surfaces and methane consumption was monitored. The study indicated a potential for the methanotroph-based technology, however, no active "industrial strain" was identified and no profitable process was developed.

### SUMMARY

It is object of the present invention a method for making a chimeric polypeptide comprising the major S-laver polypeptide of Methylomicrobium alcaliphilum sp.20Z (or a self-assembling fragment thereof) and a heterologous polypeptide or peptide, the method comprising recombinantly engineering Methylomicrobium alcaliphilum sp. 20Z to recombinantly express said chimeric polypeptide and deliver it outside of the cell where it exists in the form of an assembled monomolecular layer.

It is also object of the present invention a genetically engineered Methylomicrobium alcaliphilum sp. 20Z comprising an expression cassette which recombinantly expresses the chimeric protein comprising the major S-laver polypeptide of Methylomicrobium alcaliphilum sp.20Z (or a self-assembling fragment thereof) and a heterologous polypeptide or peptide and delivers it outside of the cell where it exists in the form of an assembled monomolecular layer.

It is also object of the invention an isolated assembled monomolecular layer comprising the chimeric protein comprising the major S-laver polypeptide of Methylomicrobium alcaliphilum sp.20Z (or a self-assembling fragment thereof) and a heterologous polypeptide or peptide.

### BRIEF DESCRIPTION OF THE DRAWINGS

The drawings set forth herein are illustrative of embodiments as provided herein and are not meant to limit the scope of the invention as encompassed by the claims.
FIG. 1A-B illustrate Lipase production in methanotrophic cultures:
FIG. 1A illustrates an image of *in vivo* activity, or intracellular production, of a lipase gene cloned into an expression vector and introduced into *Methylomicrobium sp. AP18,* as detected on rhodamine B-containing plates;
FIG. 1B illustrates an image of a Coumassie-stained polyacrylamide gel (PAAG) showing that a lipase gene expression vector showed low levels of lipase expression, e.g., the protein was not visible in lane 5; and an optimized vector with a ribosome binding site (RBS) resulted in clones with significantly higher expression, with the recombinant lipase comprising of about 1% to 2% of total cell protein, see lanes 6 to 12,
   as described in detail in Example 1, below.
FIG. 2A-B illustrate construction and expression of a vector containing a novel genetically altered microbial catalyst producing lipase:

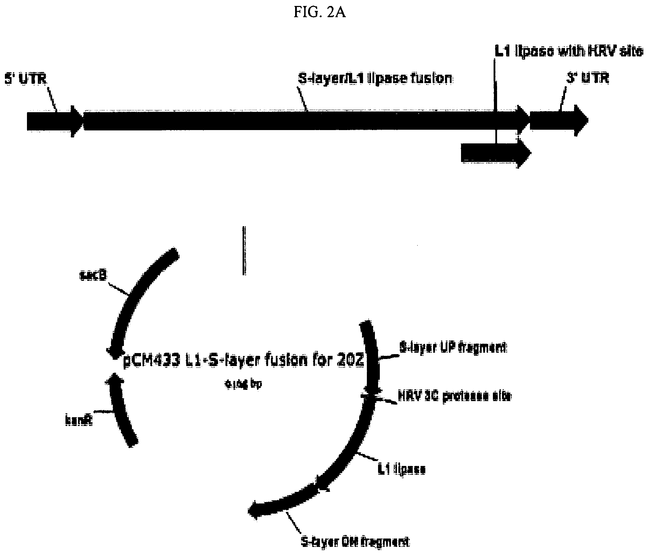

Fig. 2A, schematically illustrates in upper and lower images a lipase enzyme-encoding nucleic acid construct, and a plasmid containing this genetic construct, respectively;

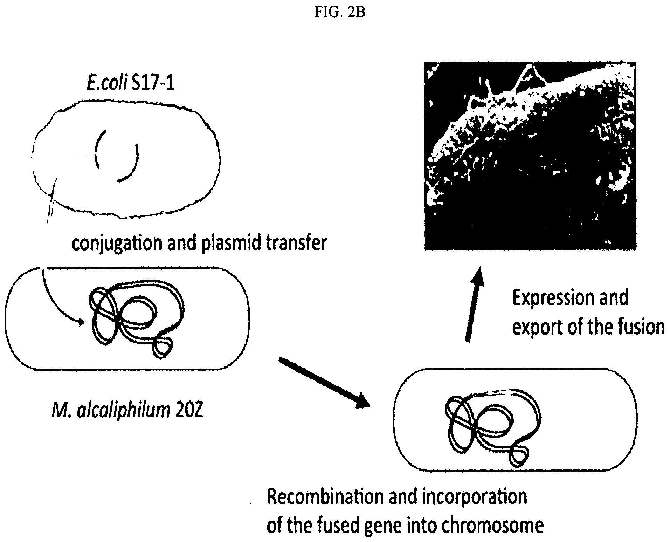

FIG. 2B schematically illustrates how this genetic construct is transferred from E. coli S 17-1 by conjugation and plasmid transfer into an *M. alcaliphilum* 20Z strain, followed by recombination and incorporation of the fused gene (the genetic construct) into the chromosome, resulting in expression and export of the fusion protein,
   as described in detail in Example 1, below.

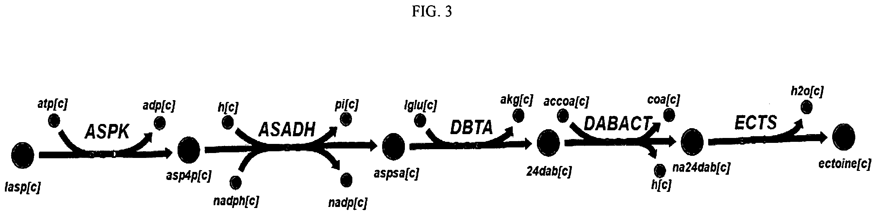

FIG. 3, schematically illustrates the ectoine biosynthesis pathway in *M. alcaliphilum* 20Z, including three specific enzymes: diaminobutyric acid (DABA) aminotransferase (EctB), DABA acetyltransferase (EctA), and ectoine synthase (EctC), as described in detail in Example 2, below.

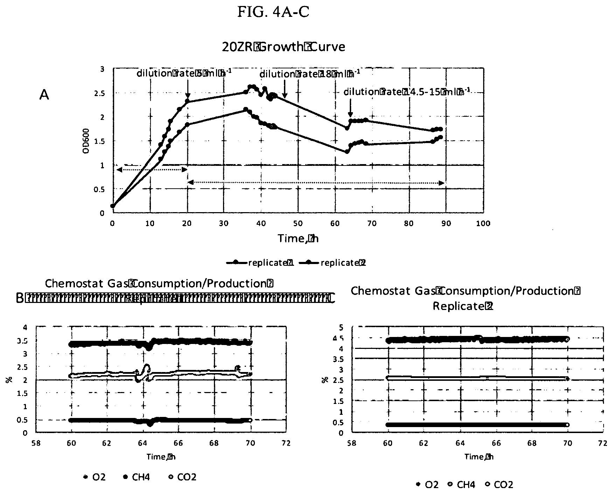

FIG. 4A-C: FIG. 4A graphically illustrates data showing the growth of *M. alcaliphilum* 20ZR in a DASBOX^{™} mini bioreactor, as batch and chemostat mode; FIG. 4B-C graphically illustrate O₂ and CH₄ consumptions and CO₂ production in steady-state for bioreactor replicate 1 (FIG. 4B) and bioreactor replicate 2 (FIG. 4C), as described in detail in Example 2, below.

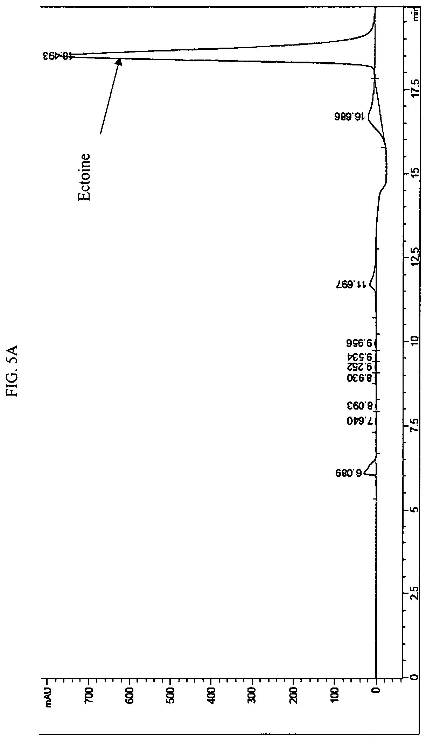

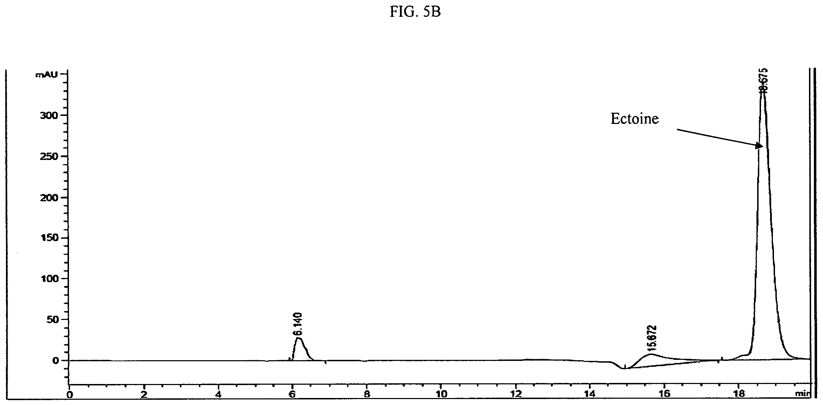

FIG. 5A-B: FIG. 5A illustrates a chromatogram of 1mM ectoine solution, and FIG. 5B illustrates a chromatogram of 20ZR cell extract, as described in detail in Example 2, below.

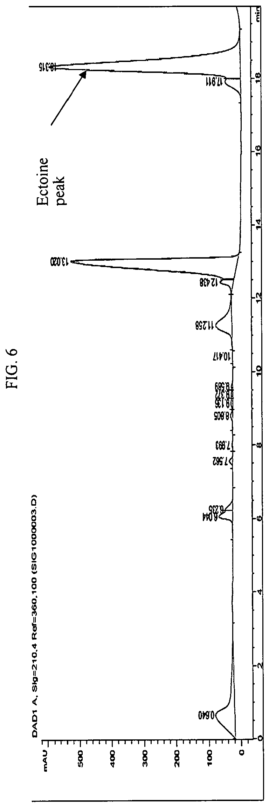

FIG. 6 illustrates a chromatogram of an HPLC analysis of 20Z^{R} wild-type as a batch culture, as described in detail in Example 2, below.

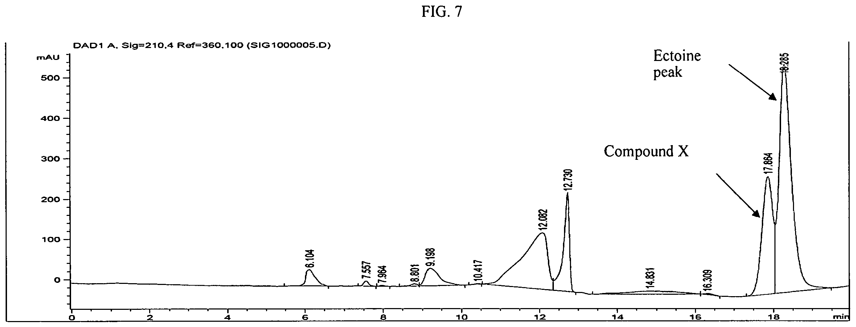

FIG. 7 illustrates a chromatogram of an HPLC analysis of 20Z^{R}ΔectR strain in batch culture, as described in detail in Example 2, below.

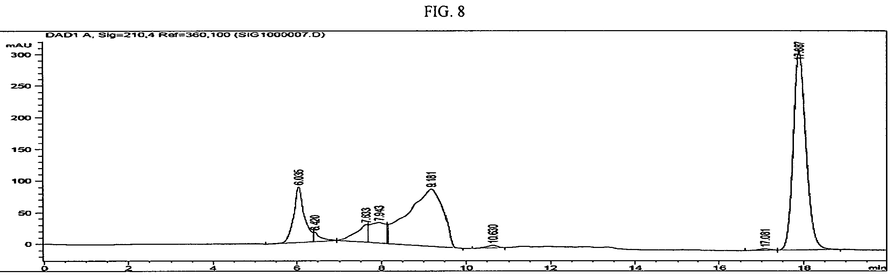

FIG. 8 illustrates a chromatogram of an HPLC analysis of 20Z^{R}ΔectRΔdoeA strain (batch culture), as described in detail in Example 2, below.

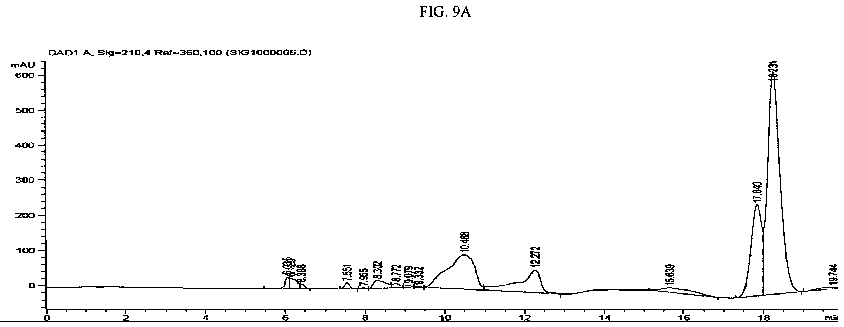

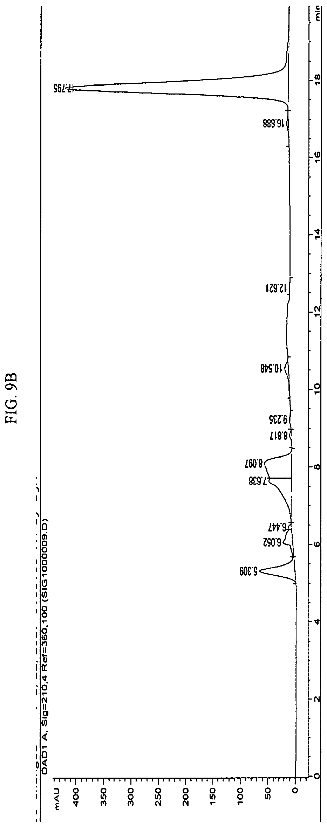

FIG. 9A-B illustrates chromatograms HPLC analyses that reveal the highest levels of ectoine: FIG. 9A illustrates HPLC analysis of HPLC analysis of 20Z::P_{SL}-L1ΔectR (in batch culture); and, FIG. 9B illustrates 20Z^{R}P_{SL}-L1ΔectR ΔdoeA strain (in batch culture), as described in detail in Example 2, below.

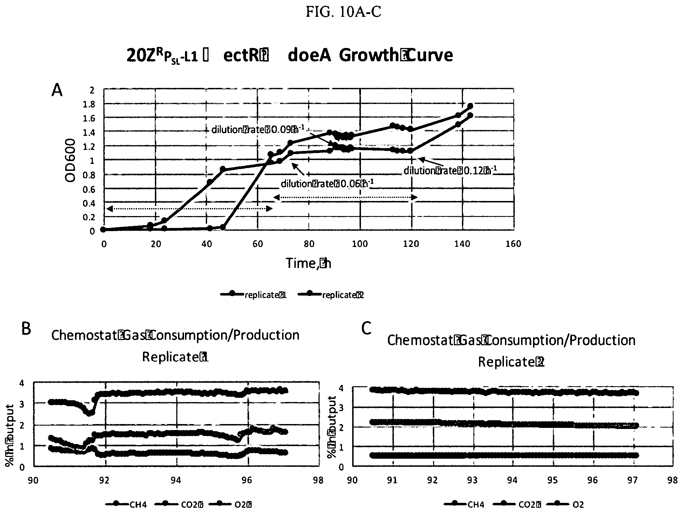

FIG. 10A-C illustrate data from the cultivation of TWC#G2-3 as performed in a DASBOX^{™} mini bioreactor, and collected data is shown as: FIG. 10A graphically illustrates growth of *M. alcaliphilum* 20Z^{R}P_{SL}-L1ΔectRΔdoeA as batch and chemostat mode; FIG. 10B-C graphically illustrate O₂ and CH₄ consumptions and CO₂ production in steady-state for bioreactor replicate 1 (FIG. 10B) and bioreactor replicate 2 (FIG. 10C), as described in detail in Example 2, below.

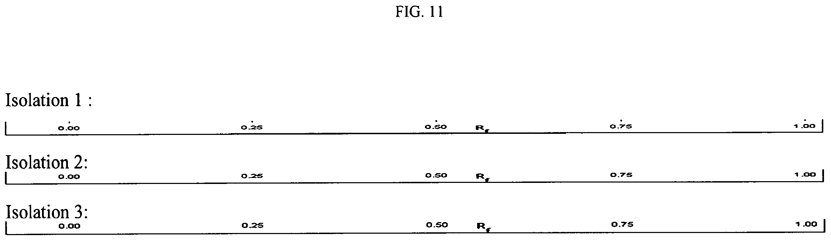

FIG. 11 illustrates LipL1 preparations, as described in detail in Example 2, below.

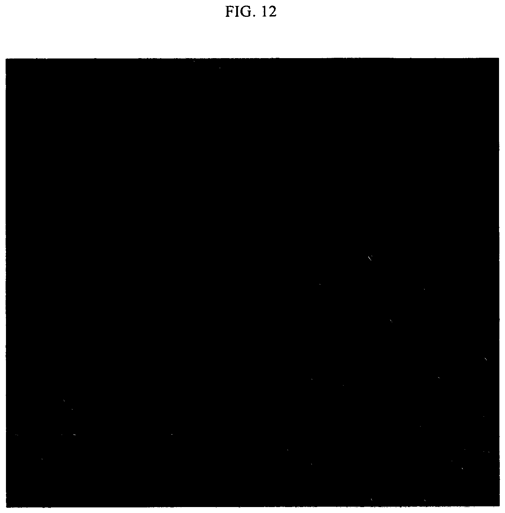

FIG. 12 illustrates an image of production of the GFP-comprising recombinant protein by the 20ZR-L1-SL strain, as described in detail in Example 3, below.

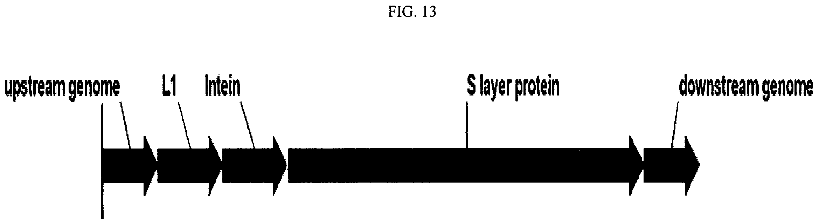

FIG. 13 schematically illustrates exemplary genetic constructs containing self-cleavable inteins, which were inserted between the lipase and the S-layer, as described in detail in Example 3, below.

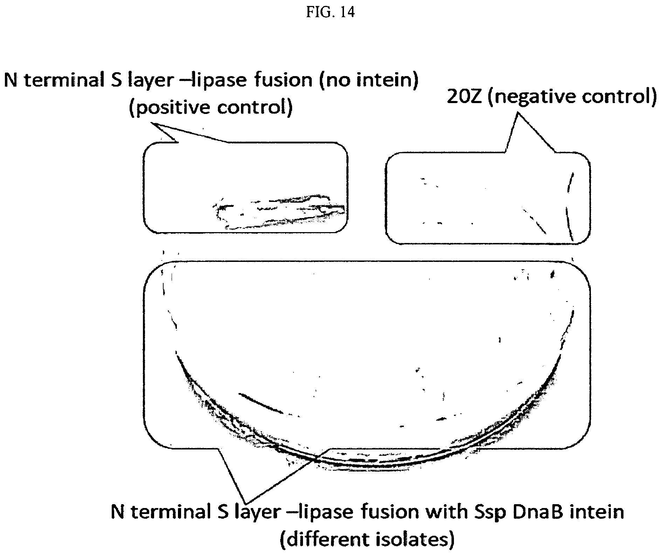

FIG. 14 illustrates an image of extracellular lipase localization and activity in a Rhodamine B assay with TWC#11 (top left of the plate), wild type (WT) (top right of the plate) and lipL- Ssp DnaB intein mutants (bottom of the plate), as described in detail in Example 3, below.

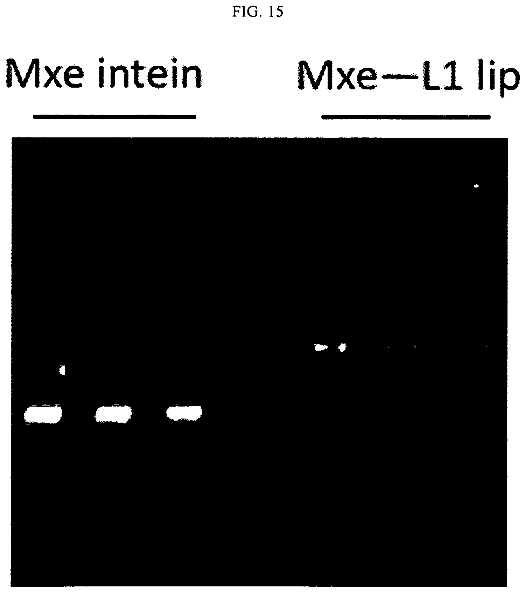

FIG. 15 illustrates an image of a gel separating amplified nucleic acid from a PCR-genotyping of TWC#G14 20ZR:: SL_{Nter}-LipL1- Mxe GyrA strain: FIG. 15 left: LipL locus, indicating that LipL-MxeGyrA- was incorporated into the genome; FIG. 15 right: LipL-S-layer locus from showing that LipL gene was incorporated in correct orientation, as LipL-MxeGyrA-S-layer, as described in detail in Example 3, below.

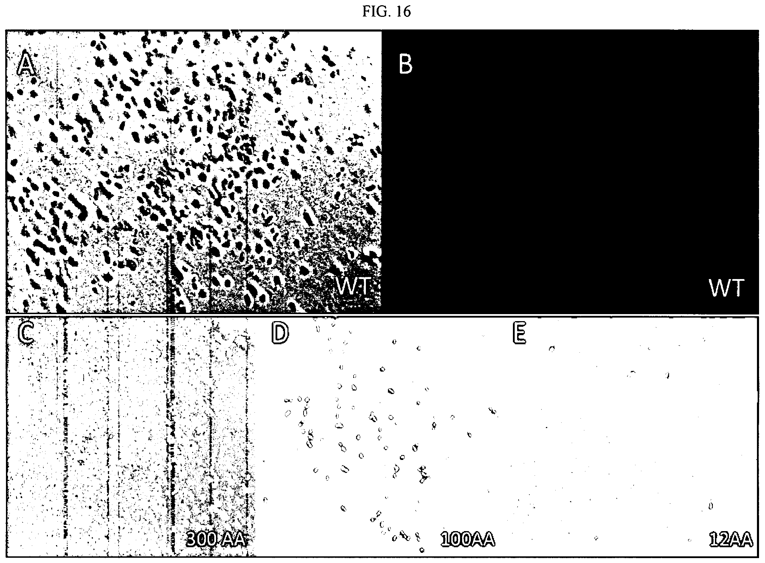

FIG. 16A-E illustrate images of cells harboring various GFP fusions: FIG. 16A-B illustrate *M. alcaliphilum* 20ZR wild type (FIG. 16A, phase; FIG. 16B, GFP ex450-490nm; em 500-550nm); FIG. 16C, 20ZR:: GFP-300Cter_{SLP}; FIG. 16D, GFP-100Cter_{SLP}; FIG. 16E, GFP-12Cter_{SLP}, as described in detail in Example 3, below.

Like reference symbols in the various drawings indicate like elements.

Reference will now be made in detail to various exemplary embodiments of the invention, examples of which are illustrated in the accompanying drawings. The following detailed description is provided to give the reader a better understanding of certain details of aspects and embodiments of the invention, and should not be interpreted as a limitation on the scope of the invention.

### DETAILED DESCRIPTION

### Chimeric polypeptides comprising an S-layer polypeptide, or self-assembling or self-aggregating fragments thereof, and a heterologous polypeptide or peptide

Notwithstanding any indication to the contrary, the claimed invention relates to an assembled monomolecular layer and to the major S-layer polypeptide of Methylomicrobium alcaliphilum sp. 20Z in accordance with the appended claims. Other embodiments or aspects are useful for understanding the invention but are not part of the claimed invention.

In alternative embodiments, provided are compositions and methods for making a chimeric polypeptide comprising an S-layer polypeptide, or self-assembling or self-aggregating fragments thereof, and a heterologous polypeptide or peptide. In alternative embodiments, the compositions and methods comprise recombinantly engineering a methylotrophic or methanotrophic bacteria to recombinantly express a chimeric polypeptide comprising an S-layer polypeptide, or self-assembling or self-aggregating fragments thereof, and a heterologous polypeptide or peptide. In alternative embodiments, the S-layer polypeptide, or self-assembling or self-aggregating fragments thereof, is engineered to be amino terminal to, internal to, or carboxy terminal to, the heterologous polypeptide or peptide.

In alternative embodiments, provided are compositions and methods comprising a recombinant or isolated chimeric S-layer polypeptide, wherein the recombinant or isolated chimeric S-layer polypeptide comprises an S-layer polypeptide, or self-assembling or self-aggregating fragments thereof, and a heterologous polypeptide or peptide. In alternative embodiments, the S-layer polypeptide, or self-assembling fragments or self-aggregating thereof, is engineered to be amino terminal to, internal to, or carboxy terminal to, the heterologous polypeptide or peptide.

Also provided are recombinant or isolated monomolecular layers comprising a chimeric S-layer polypeptide, where the S-layer polypeptide can be a self-assembling or self-aggregating fragment thereof. In alternative embodiments, provided are compositions and methods comprising recombinant methylotrophic or methanotrophic bacteria comprising assembled or self-assembled recombinant or isolated chimeric S-layer polypeptides.

Provided herein for the first time are applications of bacterial extracellular methanotrophic S-layer proteins, or self-assembling or self-aggregating fragments thereof, for the expression of heterologous proteins, including extracellular expression of a heterologous protein on the surface of a methanotrophic S-layer protein-expressing bacteria. In alternative embodiments, methanotrophic S-layers, either isolated (e.g., as described in Khmelenina VN, et al (1999) Arch. Microbiol. 172: 321-329) or as surface-expressed methanotrophic S-layers, are used as an anchor or expression vehicle for the extracellular production, expression and use of proteins, e.g., enzymes such as industrial enzymes, e.g. proteinases, lipases, amylases, celluloses, fl-glucanase, as well as for their use as enzymatic systems for bioremediation and bio-mitigations, e.g. dehalogenases and peroxidases, and protein-based biopharmaceuticals.

We identified the gene encoding the major S-layer protein in *M. alcaliphilum* sp. 20Z using quantitative proteomics on purified S-layer preparations. The S-layer protein appears to be the main cellular protein, comprising up to 20% of total cellular protein.

In alternative embodiments of compositions and methods as provided herein, chimeric proteins are delivered and expressed outside of the bacterial cell, e.g., chimeric proteins as provided herein are expressed extracellularly can be completely or partially exposed to an extracellular milieu.

We developed a protocol for genetic alterations of the S-layers for heterologous expression of proteins on a bacterial cell surface. Overview of the approach is shown in FIG. 2.

### S-layer polypeptides, or self-assembling or self-aggregating fragments thereof

Exemplary S-layer polypeptides, or self-assembling or self-aggregating fragments thereof, can comprise or consist of:
SEQ ID NO:8 
GANNQVAALQTAAGGAFDGTFFDVLSNFTVEASFEVLSQFDPETTLFVANPIV
EDVNFDIVRDVNGDVTSVSVSGGSSLGFAQSDAGFQELLEAGQVTEVVFENV
GSLNSILVSGNFVGSYDAGGIFYESTFEFGANAGSVAEGVGTDGNIFTIAEFTA
GAAASDILDFTAMPVDNTNTAPATGHEFIAVGTEASIGDDATIIVFTAGVAAD
AATIVTQFADGAGDFRSADATARNADFAIDSQLIFLIDDGAGNTGVWYWDDT
VGAVGDGIVDADELSQIAQLTGVVTAELTVDNFVLA,
SEQ ID NO:9 
TIIVFTAGVAADAATIVTQFADGAGDFRSADATARNADFAIDSQLIFLIDDGAG
NTGVWYWDDTVGAVGDGIVDADELSQIAQLTGVVTAELTVDNFVLA,
SEQ ID NO:10
   AGNTGVWYWDDTVGAVGDGIVDADELSQIAQLTGVVTAELTVDNFVLA,
SEQ ID NO:11
   VGAVGDGIVDADELSQIAQLTGVVTAELTVDNFVLA,
SEQ ID NO:12
   TAELTVDNFVLA, or
the S-layer polypeptide as encoded by the nucleic acid sequence of SEQ ID NO:5, SEQ ID NO:7 and/or SEQ ID NO:7, as indicated below.

Exemplary S-layer polypeptide self-assembling or self-aggregating fragments thereof also can comprise or consist of any self-assembling fragment, which can be readily identified by routine screening of an S-layer polypeptide.

Exemplary S-layer polypeptides or self-assembling or self-aggregating fragments thereof also comprise S-layer polypeptide sequences as described herein but comprising at least one amino acid residue conservative substitution, wherein optionally the at least one conservative substitution comprises replacement of an aliphatic amino acid with another aliphatic amino acid; replacement of a serine with a threonine or vice versa; replacement of an acidic residue with another acidic residue; replacement of a residue bearing an amide group with another residue bearing an amide group; exchange of a basic residue with another basic residue; or, replacement of an aromatic residue with another aromatic residue, or a combination thereof, and optionally the aliphatic residue comprises Alanine, Valine, Leucine, Isoleucine or a synthetic equivalent thereof; the acidic residue comprises Aspartic acid, Glutamic acid or a synthetic equivalent thereof; the residue comprising an amide group comprises Aspartic acid, Glutamic acid or a synthetic equivalent thereof; the basic residue comprises Lysine, Arginine or a synthetic equivalent thereof; or, the aromatic residue comprises Phenylalanine, Tyrosine or a synthetic equivalent thereof.

### Recombinant methylotrophic or methanotrophic bacteria to express heterologous proteins

In alternative embodiments, provided are compositions and methods using a *M. alcaliphilum* sp. 20Z, for ectoine ((4S)-2-methyl-1,4,5,6-tetrahydropyrimidine-4-carboxylic acid), for the production or synthesis of a protein, e.g., an ectoine (1,4,5,6-tetrahydro-2-methyl-4-pyrimidinecarboxylic acid), or an enzyme, e.g., a lipase.

### Methanotrophic S-Layers and S-Layer-Based Enzyme Immobilization

In alternative embodiments, compositions and methods as provided herein use S-layers, which are a well-recognized microbial product with very broad biotechnological applications, see e.g., Egelseer et al., 2009, NanoBioTechnology (Shoseyov O & Levy I, eds), pp. 55-86. Humana Press, Totowa, NJ; or Egelseer et al., The Encyclopedia of Industrial Biotechnology: Bioprocess, Bioseparation, and Cell Technology, Vol. 7 (Flickinger MC, ed.), pp. 4424-4448. John Wiley & Sons, Inc., Hoboken, NJ. In alternative embodiments, compositions and methods as provided herein comprise the construction of fusion proteins comprising S-layer proteins with attached enzymes (or other proteins) for the production of immobilized biocatalysts. In alternative embodiments, S-layers derived from *Methylomicrobium alcaliphilum* 20Z are used. Formation of S-layers has been observed in all tested *Methylomicrobium* species. In alternative embodiments, S-layer proteins are positioned carboxy terminal to the attached protein, although they can also be internal or amino terminus positioned.

We identified the gene encoding the major S-layer protein in M. *alcaliphilum* sp. 20Z using quantitative proteomics on purified S-layer preparations; see Example 2, below. The S-layer protein appears to be the main cellular protein, comprising up to 20% of total cellular protein. In alternative embodiments, compositions and methods as provided herein use S-layers as an efficient cellular system to deliver proteins outside of the cell. In alternative embodiments, this system is also used to produce biological filters or purification systems, and enzymatic membranes.

### Generating and Manipulating Nucleic Acids

In alternative embodiments, nucleic acids used to practice methods as provided herein, or to make compositions or recombinant bacteria as provided herein, are made, isolated and/or manipulated by, e.g., cloning and expression of cDNA libraries, amplification of message or genomic DNA by PCR, and the like. The nucleic acids and genes used to practice this invention, including DNA, RNA, iRNA, antisense nucleic acid, cDNA, genomic DNA, vectors, viruses or hybrids thereof, can be isolated from a variety of sources, genetically engineered, amplified, and/or expressed/ generated recombinantly. Recombinant polypeptides generated from these nucleic acids can be individually isolated or cloned and tested for a desired activity. Any recombinant expression system or gene therapy delivery vehicle can be used, including e.g., viral (e.g., AAV constructs or hybrids) bacterial, fungal, mammalian, yeast, insect or plant cell expression systems or expression vehicles.

Alternatively, nucleic acids used to practice methods as provided herein, or to make compositions or recombinant bacteria as provided herein, can be synthesized *in vitro* by well-known chemical synthesis techniques, as described in, e.g., Adams (1983) J. Am. Chem. Soc. 105:661; Belousov (1997) Nucleic Acids Res. 25:3440-3444; Frenkel (1995) Free Radic. Biol. Med. 19:373-380; Blommers (1994) Biochemistry 33:7886-7896; Narang (1979) Meth. Enzymol. 68:90; Brown (1979) Meth. Enzymol. 68:109; Beaucage (1981) Tetra. Lett. 22:1859; U.S. Patent No. 4,458,066.

Techniques for the manipulation of nucleic acids used to practice methods as provided herein, or to make compositions or recombinant bacteria as provided herein, such as, e.g., subcloning, labeling probes (e.g., random-primer labeling using Klenow polymerase, nick translation, amplification), sequencing, hybridization and the like are well described in the scientific and patent literature, see, e.g., Sambrook, ed., MOLECULAR CLONING: A LABORATORY MANUAL (2ND ED.), Vols. 1-3, Cold Spring Harbor Laboratory, (1989); CURRENT PROTOCOLS IN MOLECULAR BIOLOGY, Ausubel, ed. John Wiley & Sons, Inc., New York (1997); LABORATORY TECHNIQUES IN BIOCHEMISTRY AND MOLECULAR BIOLOGY: HYBRIDIZATION WITH NUCLEIC ACID PROBES, Part I. Theory and Nucleic Acid Preparation, Tijssen, ed. Elsevier, N.Y. (1993).

Another useful means of obtaining and manipulating nucleic acids used to practice methods as provided herein, or to make compositions or recombinant bacteria as provided herein, is to clone from genomic samples, and, if desired, screen and re-clone inserts isolated or amplified from, e.g., genomic clones or cDNA clones. Sources of nucleic acid used in the methods of the invention include genomic or cDNA libraries contained in, e.g., mammalian artificial chromosomes (MACs), see, e.g., U.S. Patent Nos. 5,721,118; 6,025,155; human artificial chromosomes, see, e.g., Rosenfeld (1997) Nat. Genet. 15:333-335; yeast artificial chromosomes (YAC); bacterial artificial chromosomes (BAC); P1 artificial chromosomes, see, e.g., Woon (1998) Genomics 50:306-316; P1-derived vectors (PACs), see, e.g., Kern (1997) Biotechniques 23:120-124; cosmids, recombinant viruses, phages or plasmids.

In alternative embodiments, a heterologous peptide or polypeptide joined or fused to a protein made by a method or a recombinant bacteria as provided herein can be an N-terminal identification peptide which imparts a desired characteristic, such as fluorescent detection, increased stability and/or simplified purification. Peptides and polypeptides made by a method or a recombinant bacteria as provided herein can also be synthesized and expressed as fusion proteins with one or more additional domains linked thereto for, e.g., producing a more immunogenic peptide, to more readily isolate a recombinantly synthesized peptide, to identify and isolate antibodies and antibody-expressing B cells, and the like. Detection and purification facilitating domains include, e.g., metal chelating peptides such as polyhistidine tracts and histidine-tryptophan modules that allow purification on immobilized metals, protein A domains that allow purification on immobilized immunoglobulin, and the domain utilized in the FLAGS extension/affinity purification system (Immunex Corp, Seattle WA). The inclusion of a cleavable linker sequences such as Factor Xa or enterokinase (Invitrogen, San Diego CA) between a purification domain and the motif-comprising peptide or polypeptide to facilitate purification. For example, an expression vector can include an epitope-encoding nucleic acid sequence linked to six histidine residues followed by a thioredoxin and an enterokinase cleavage site (see e.g., Williams (1995) Biochemistry 34:1787-1797; Dobeli (1998) Protein Expr. Purif. 12:404-414). The histidine residues facilitate detection and purification while the enterokinase cleavage site provides a means for purifying the epitope from the remainder of the fusion protein. Technology pertaining to vectors encoding fusion proteins and application of fusion proteins are well described in the scientific and patent literature, see e.g., Kroll (1993) DNA Cell. Biol., 12:441-53.

Nucleic acids or nucleic acid sequences used to practice embodiments as provided herein can be an oligonucleotide, nucleotide, polynucleotide, or to a fragment of any of these, to DNA or RNA of genomic or synthetic origin which may be single-stranded or double-stranded and may represent a sense or antisense strand, to peptide nucleic acid (PNA), or to any DNA-like or RNA-like material, natural or synthetic in origin. Compounds use to practice this invention include "nucleic acids" or "nucleic acid sequences" including oligonucleotide, nucleotide, polynucleotide, or any fragment of any of these; and include DNA or RNA (e.g., mRNA, rRNA, tRNA, iRNA) of genomic or synthetic origin which may be single-stranded or double-stranded; and can be a sense or antisense strand, or a peptide nucleic acid (PNA), or any DNA-like or RNA-like material, natural or synthetic in origin, including, e.g., iRNA, ribonucleoproteins (e.g., e.g., double stranded iRNAs, e.g., iRNPs). Nucleic acids or nucleic acid sequences used to practice embodiments as provided herein include nucleic acids or oligonucleotides containing known analogues of natural nucleotides. Nucleic acids or nucleic acid sequences used to practice embodiments as provided herein include nucleic-acid-like structures with synthetic backbones, see e.g., Mata (1997) Toxicol. Appl. Pharmacol. 144:189-197; Strauss-Soukup (1997) Biochemistry 36:8692-8698; Samstag (1996) Antisense Nucleic Acid Drug Dev 6:153-156. Nucleic acids or nucleic acid sequences used to practice embodiments as provided herein include "oligonucleotides" including a single stranded polydeoxynucleotide or two complementary polydeoxynucleotide strands that may be chemically synthesized. Compounds use to practice this invention include synthetic oligonucleotides having no 5' phosphate, and thus will not ligate to another oligonucleotide without adding a phosphate with an ATP in the presence of a kinase. A synthetic oligonucleotide can ligate to a fragment that has not been dephosphorylated.

In alternative aspects, methods and recombinant bacteria as provided herein comprise use of "expression cassettes" comprising a nucleotide sequences capable of affecting expression of the nucleic acid, e.g., a structural gene or a transcript (e.g., encoding an S-layer protein, and/or an enzyme such as a lipase or a ectoine) in a host compatible with such sequences, such as e.g., methylotrophic and methanotrophic cells such as *Methylococcus, Methylothermus,* and *Methylomicrobium* bacterial cells. Expression cassettes can include at least a promoter operably linked with the polypeptide coding sequence or inhibitory sequence; and, in one aspect, with other sequences, e.g., transcription termination signals. Additional factors necessary or helpful in effecting expression may also be used, e.g., enhancers.

In alternative aspects, expression cassettes used to practice embodiments as provided herein also include plasmids, expression vectors, recombinant viruses, any form of recombinant "naked DNA" vector, and the like. In alternative aspects, a "vector" used to practice embodiments as provided herein can comprise a nucleic acid that can infect, transfect, transiently or permanently transduce a cell. In alternative aspects, a vector used to practice embodiments as provided herein can be a naked nucleic acid, or a nucleic acid complexed with protein or lipid. In alternative aspects, vectors used to practice embodiments as provided herein can comprise viral or bacterial nucleic acids and/or proteins, and/or membranes (e.g., a cell membrane, a viral lipid envelope, etc.). In alternative aspects, vectors used to practice embodiments as provided herein can include, but are not limited to replicons (e.g., RNA replicons, bacteriophages) to which fragments of DNA may be attached and become replicated. Vectors thus include, but are not limited to RNA, autonomous self-replicating circular or linear DNA or RNA (e.g., plasmids, viruses, and the like, see, e.g., U.S. Patent No. 5,217,879), and can include both the expression and non-expression plasmids. In alternative aspects, the vector used to practice embodiments as provided herein can be stably replicated by the cells during mitosis as an autonomous structure or can be incorporated within the host's genome.

In alternative aspects, "promoters" used to practice this invention include all sequences capable of driving transcription of a coding sequence in a bacterial cell, e.g., a methylotrophic or methanotrophic bacterial cell. Thus, promoters used in the constructs of the invention include cis-acting transcriptional control elements and regulatory sequences that are involved in regulating or modulating the timing and/or rate of transcription of a gene. For example, a promoter used to practice this invention can be a cis-acting transcriptional control element, including an enhancer, a promoter, a transcription terminator, an origin of replication, a chromosomal integration sequence, 5' and 3' untranslated regions, or an intronic sequence, which are involved in transcriptional regulation. These cis-acting sequences typically interact with proteins or other biomolecules to carry out (turn on/off, regulate, modulate, etc.) transcription.

### Bacterial Growth Conditions

Any set of known growth conditions can be used to practice embodiments as provided herein, for example, as described in US 2016-0237398 A1, or WO/2015/058179; exemplary growth conditions and parameters are described in Example 1 and Example 2, below. Any known growth conditions for culturing methylotrophic and methanotrophic cells such as *Methylococcus, Methylothermus,* and *Methylomicrobium* bacterial cells can be used.

The invention will be further described with reference to the following examples; however, it is to be understood that the invention is not limited to such examples.

### EXAMPLES

### Example 1: Exemplary Methods and Compositions

This example provides exemplary methods for making compositions and bacterial cells as provided herein.

### Methanotrophic strain best suited for biotechnological exploration.

Two methanotrophic cultures were established as the most promising industrial strains: *Methylomicrobium alcaliphilum* sp. 20Z and *Methylomicrobium buryatenses* 5G (see, e.g., Ojala et al., 2011; Kalyuzhnaya et al., 2015; Puri et al., 2015; Strong et al., 2016). While *M. buryatenses* 5G represents a fast-growing methanotroph (Td=3h), *M. alcaliphilum* sp. 20Z was found to be more stable at high-cell density. Furthermore, the latter strain has a greater potential for accumulation of extractable products (ectoine, glutamate, sucrose). Based on those characteristics, *M*. *alcaliphilum* sp. 20Z was selected.

Genomes of both *M. alcaliphilum* sp. 20Z and *M. buryatenses* 5G were sequenced (see e.g., Vuilleumier et al., 2012). Genetic tools for efficient metabolic engineering of the strains were developed or optimized (see e.g., Ojala et al., 2011; Puri 2015; Henard et al., 2016). The current toolbox includes: vectors for gene knockouts (incorporated via bi-parental mating or electroporation); vectors for heterologous expression with low, intermediate and high levels of expression; and vectors with tunable promoters. Provided is a whole-genome reconstruction of the *M. alcaliphilum* sp. 20Z metabolic network, which is refined via metabolomics on cells grown in liquid culture, providing a computation framework for additional optimization of metabolic pathways in producing traits.

### Methanotrophic S-Layers an S-Layer-Based Enzyme Immobilization

Here we describe use of S-layers as an efficient cellular system to deliver proteins outside of the cell. In alternative embodiments, this system is also used to produce biological filters and enzymatic membranes, or purification systems.

We identified the gene encoding the major S-layer protein in M. *alcaliphilum* sp. 20Z using quantitative proteomics on purified S-layer preparations. The S-layer protein appears to be the main cellular protein, comprising up to 20% of total cellular protein.

### Lipase production in methanotrophic cultures.

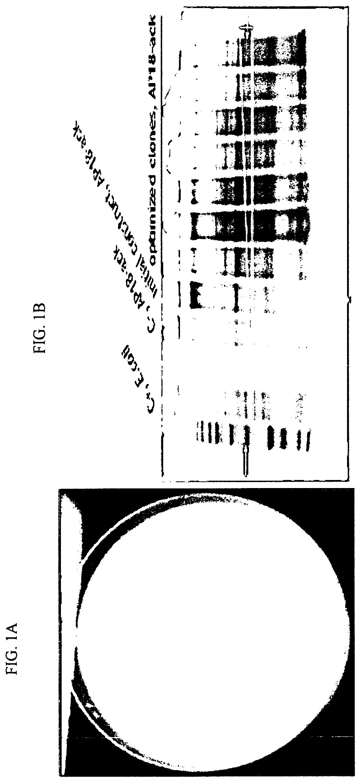

Lipase production by *Bacillus stearothermophilus* L1 [Kim et al. 2000] is optimal at 60°C to 65°C and pH 9 to pH 11 [Kim et al. 1998]. This lipase has been shown to have a 2 to 4 times higher activity for saturated fatty acids compared to monounsaturated ones. This makes L1 lipase a good candidate for hydrolysis of solid lipids like beef tallow and palm oil which are known to be difficult targets for currently used lipases. L1 lipase gene was codon optimized for efficient expression in methanotrophic host. The gene was synthetized and cloned into an expression vector and introduced into *Methylomicrobium sp. AP18* for intracellular production; it's *in vivo* activity was detected on rhodamine B-containing plates (Fig. 1A). However, this construct showed low levels of lipase expression, i.e., the protein was not visible on Coumassie-stained polyacrylamide gel (PAAG) (Fig 1B, lane 5).

In order to increase the expression, optimization of its ribosome binding site using *in-house* protocol was performed resulting in selection of clones with significantly higher expression (L1 comprising of about 1% to 2% of total cell protein (Fig 1B, lanes 6 to 12)).

### Construction of a novel genetically altered microbial catalyst producing lipase (up to 10% of CDW)

In order to further increase lipase production and simultaneously simplify its purification, an *M. alcaliphilum* 20Z strain expressing L1 lipase extracellularly as a fusion with S-layer protein is constructed. The fusion protein is introduced into the *M. alcaliphilum* 20Z chromosome using its native genetic elements to ensure high expression and proper extracellular localization of the fusion.

To facilitate lipase isolation, a site for HRV 3C protease is introduced between the S-layer and lipase polypeptides allowing the fusion protein to be cleaved with HRV 3C protease to release functional L1 lipase into solution, a genetic construct encoding this fusion protein is schematically illustrated in Fig. 2A, upper and lower images.

A plasmid containing this genetic construct is transferred from E. coli S17-1 by conjugation and plasmid transfer into an *M. alcaliphilum* 20Z strain, followed by recombination and incorporation of the fused gene (the genetic construct) into the chromosome, resulting in expression and export of the fusion protein, as schematically illustrated in FIG. 2B.

### Methods.

The genetic manipulation includes the following set of steps:
1. PCR amplification of the codon optimized L1-gene and pCM433 vector;
2. PCR amplification of the S-layer upstream and downstream flanks. All reactions are done with a Q5 high-fidelity DNA polymerase;
3. Gibson assembly and transformation into *E.coli* NEB 5-alpha are performed using NEBuilder HiFi^{™} DNA assembly kit (NEBlabs);
4. Selected clones are validated by PCR (with Tag-polymerase, Invitrogen) and sequenced (at Eton Bioscience Center);
5. Validated plasmids are subcloned into *E.coli* S17-1 via transformation;
6. Biparental mating with *M. alcaliphilum* and clone selection is set up as described by Ojala et al. [2011];
7. Genotype characteristics is validated by PCR;
8. Phenotype characteristics of new traits is evaluated via scanning electron microscopy (SEM), lipase activity (rhodamine B assay) and SDS-PAAG electrophoresis.

### Example 2: Exemplary Methods and Compositions

This example provides exemplary methods for making compositions and bacterial cells as provided herein, and practicing methods as provided herein.

Provided herein are new mitigation strategies for effective conversion of atmospheric greenhouse gases (e.g., CO₂ or methane) to next generation chemicals which are a new technology for the reduction/stabilization of global warming. In alternative embodiments, methods provided herein comprise use of biological systems (microbial cells or enzymes) as catalysts for conversion of e.g., CO₂ or methane.

### Methanotrophic strain best suited for biotechnological exploration

Two methanotrophic cultures were established as the most promising industrial strains: *Methylomicrobium alcaliphilum* sp. 20Z and *Methylomicrobium buryatenses* 5G [Ojala et al., 2011; Kalyuzhnaya et al., 2015; Puri et al., 2015; Strong et al., 2016]. While *M. buryatenses* 5G represents a fast-growing methanotroph (Td=3h), *M. alcaliphilum* sp. 20Z was found to be more stable at high-cell density. Furthermore, the latter strain has a greater potential for accumulation of extractable products (ectoine, glutamate, sucrose). Based on those characteristics, *M*. *alcaliphilum* sp. 20Z was selected.

Genomes of both *M. alcaliphilum* sp. 20Z and *M. buryatenses* 5G were sequenced (see e.g., Vuilleumier et al., 2012). Genetic tools for efficient metabolic engineering of the strains were developed or optimized (see e.g., Ojala et al., 2011; Puri 2015; Henard et al., 2016). The current toolbox includes: vectors for gene knockouts (incorporated via bi-parental mating or electroporation); vectors for heterologous expression with low, intermediate and high levels of expression; and vectors with tunable promoters. Provided is a whole-genome reconstruction of the *M. alcaliphilum* sp. 20Z metabolic network, which is refined via metabolomics on cells grown in liquid culture, providing a computation framework for additional optimization of metabolic pathways in producing traits.

### Ectoine: commercial potential and production in Methylomicrobium alcaliphilum sp. 20Z.

Provided herein are methods for making ectoine ((4S)-2-methyl-1,4,5,6-tetrahydropyrimidine-4-carboxylic acid), which is a well-known microbial compatible solute. Ectoine can be used as a chemical chaperone for industrial enzymes or pharmaceuticals, a cryoprotectant, a hydrator in skin-care products, a cell stabilizer for medical treatments, and a crop-protecting agent [Graf et al., 2008; Pastor et al., 2010].

*Methylomicrobium alcaliphilum* 20Z copes with high salinity in its growth medium by accumulating ectoine (up to 8% CDW), glutamate, and sucrose (up to 12% CDW) as major osmoprotective compounds. This strain was used as the most promising culture for ectoine production, see e.g., Totsenko et al, 2005. The ectoine biosynthesis pathway in *M. alcaliphilum* 20Z is similar to the pathway employed by halophilic/halotolerant heterotrophs and involves three specific enzymes: diaminobutyric acid (DABA) aminotransferase (EctB), DABA acetyltransferase (EctA), and ectoine synthase (EctC) (see e.g., Reshetnikov et al., 2011), as illustrated in FIG. 3. The ectoine biosynthetic gene cluster is organized as one operon (*ectABCask*) and is controlled by a negative transcriptional regulator (EctR1, marR-family). The EctR1 protein represses the expression of the *ectABC-ask* operon from the ectApi promoter.

Baseline parameters and initial rates/titer of ectoine production for the wild strain (or wild type, WT) include: biomass yield (Y: 0.46), growth rate (0.09 h-1), O₂/substrate ratios (1.54), ectoine titer (1.9% DCW).

### Strains lacking the ectR-regulator and expressing LipL1 were constructed

A set of strains lacking the ectR-regulator and expressing LipL1 were constructed. The strain lacking the ectR-regulator showed an ectoine titer similar to the WT; however, the strain demonstrated overproduction of a compound X, which was identified as a product of ectoine degradation. The deletion of the *doeA*-gene encoding ectoine hydrolase in the ΔectR background (Strain 20Z^{R}ΔectRΔdoeA) eliminated compound X accumulation and led to an increased production of ectoine (2.4% DCW). A LipL1 expression plasmid (P_{SL}-L1 construct) was subsequently incorporated into 20Z^{R}P_{SL}-L1ΔectR (to create TWC#G2) and 20Z^{R}P_{SL}-L1ΔectRΔdoeA (to create TWC#G2-3) which express LipL1. The specific activity of methanotrophic lipase is 1.2 U g⁻¹ CDW.

The growth rates of the strains TWC#G2, TWC#G2-2 and TWC#G2-3 are similar to WT. The strains TWC#G2-2 and TWC#G2-3 showed elevated ectoine (2.4% and 3.1% DCW, respectively), which corresponds to a production rates of 2.2 and 2.8 mg g⁻¹ CDW h⁻¹, respectively. The chemostat culture of TWC#G2-3 displays similar properties to WT growth kinetics and shows ectoine production as 3.3 mg g⁻¹ CDW h⁻¹. Thus TWC#G2-3 shows 1.7/1.8-fold improvement. The specific activity of methanotrophic lipase in TWC#G2-3 is 1.2 U g⁻¹ CDW.

Expression and purification of LipL1 protein. LipL was expressed and purified. Twelve mg of the protein with the specific activity of 589 U/mg were produced.

**Table 1. List of exemplary genetic modifications in M. alcaliphilum sp. 20Z^{R}.**

| Genetic alteration | Strain | Locus tag | Phenotype | IP/publication |
|---|---|---|---|---|
| Deletion: Transcriptional regulator MarR family | ΔectR::kan | MALCv4_32 51 | No growth defects. Overexpression of the ectABC-ask operon. | Mustakhimov et al., 2010 |
| Deletion: Sucrose-phosphate synthase | Δsps::kan | MALCv4_06 14 | No accumulation of sucrose is observed. No growth defects | WO20150581 79 A1 |
| Deletion: Glycogen synthase 1 | Δglg1::kan | MALCv4_35 07 | Glycogen accumulation is reduced (10% of WT). No growth defects | WO20150581 79 A1 |
| | | MALCv4_35 08 | | |
| Deletion: Glycogen synthase cluster 2 | Δglg2::kan | MALCv4_35 02. | Glycogen accumulation is reduced (5% of WT). No growth defects | WO20150581 79 A1 |
| | | MALCv4_35 03 | | |
| | | MALCv4_35 04 | | |
| Deletion: Amylosucrose | Δams::kan | MALCv4_06 17 | Significant decrease in intracellular glycogen accumulation and increase (15-20%) in sucrose accumulation. No growth defects | WO20150581 79 A1 |
| Deletion: EPS biosynthesis | Δeps::kan | MALCv4_06 18-MALCv4_06 19 | TBD | New |
| Deletion: ectoine hydrolase | ΔdoeA | MALCv4_32 46 | The strain was constructed in 20Z^{R} ΔectR background | New |
| Overexpression of lipase | Trait TWC#G1 | | No growth defects. Culture displays lipase activity (1.2 U g-1 DCW) | New |
| | 20Z^{R}:: SL_{Cter}-LipL1 | | | |
| EctR deletion | 20Z^{R} ΔectR | | Unmarked 20Z^{R} ΔectR strain was constructed. No growth defects. Accumulation of products of ectoine degradation. No/mild increase in ectoine accumulation. | New |
| EctR deletion and overexpression of lipase | Trait TWC#G2 | | No growth defects. Accumulation of products of ectoine degradation. No/mild increase in ectoine accumulation. | New |
| | 20Z^{R} ΔectR:: SL_{Cter}-LipL1 | | | |
| EctR deletion and DoeA deletion | Trait TWC#G2-2 | | No growth defects. Increase production of ectoine (1.6 fold increase). | New |
| | 20Z^{R} ΔectR ΔdoeA | | | |
| EctR deletion and overexpression of lipase | Trait TWC#G2-3 | | No growth defects. Increase production of ectoine (1.7 fold increase). Culture displays lipase activity (1.2 U g-1 DCW). | New |
| | 20Z^{R}:: SL_{Cter}-LipL1 | | | |
| | ΔectR ΔdoeA (C-term) | | | |
| Overexpression of Icl/ms | Trait TWC#G2-4 | | Improved growth (25% higher growth rate). Increased production of ectoine. | New, related to ectoine. |
| | 20Z^{R}::Pₕₚₛ-icl-ms | | | |
| Overexpression of icl/ms | Trait TWC#G2-v5 and v6 | | improved ectoine production (5.2%). No growth defects. | New |
| | 20Z^{R} ΔectR ΔdoeA::Pₕₚₛ-icl-ms | | | |
| Overexpression of ectoine biosynthesis | Traits TWC#G3 - TWC#G5 | | No growth defects. 6.2% DCW ectoine | New |
| | 20Z^{R} :: Phps-ectABC | | | |
| | 20Z^{R}20Z^{R} SL_{Cter}-LipL1ΔectR ΔdoeA:: Pₒₚₜ-ectABC | | | |
| Multiple deletions | TWC#G6-TWC#10 | | Traits with improved ectoine production | New |
| | 20Z^{R} SL_{Cter}-LipL1 ΔectR | | | |
| | Δsps Δglg1 Δglg2 Δeps | | | |
| | ΔdoeA:: Pₒₚₜ-ectABC-icl-ms | | | |
| Multiple deletions | Traits TWC#20 | | Traits with improved ectoine production | New |
| | 20Z^{R}ΔectR Δsps Δglg1 | | | |
| | Δglg2 Δeps ΔdoeA:: Pₒₚₜ-ectABC-icl-ms | | | |
| Expression of LipL from N-ter | TWC#G11 20Z^{R}:: SL_{Nter}-LipL1 | | Lipase protein is expressed and excreted | New |
| Multiple deletions & Overexpression of lipase and ectoine | TWC#G12 | | Terminated. New system for LipL expression should be constructed. | New |
| | 20Z^{R} SL_{Cter}-LipL1 ΔectR | | | |
| | Δsps Δglg1 Δglg2 Δeps ΔdoeA :: Pₒₚₜ-ectABC::icl-ms::SL_{Nter}-LipL1 | | | |
| Expression of LipL from N-ter with intein | TWC#G13 20Z^{R}:: SL_{Nter}-LipL1- Ssp DnaB mini-intein | | No growth defects. LipL accumulates in cytosol mostly. | New |
| Expression of LipL from N-ter | TWC#G14 20ZR:: SL_{Nter}-LipL1- Mxe GyrA | | No growth defects. | New |
| Expression of GFP with SLP C-term fusion | 20ZR:: GFP-12Cter_{SLP} | | Protein is expressed and excreted into growth medium | New |

### Initial cultivation parameters for M. alcaliphilum 20Z^{R} in batch cultures.

*Strain and growth media: M. alcaliphilum* 20Z^{R} cells were grown using modified P media (g/L): KNO₃, 1; MgSO₄ × 7H₂O, 0.2; CaCl₂ × 2H₂O, 0.02; NaCl, 30; trace solution, 1ml/L (Table 2); and supplemented with 20 ml/L of phosphate solution (5.44 g KH₂PO₄; 5.68 g Na₂HPO₄) and 20 ml/L of 1M carbonate buffer.

**Table 2. Trace solution composition.**

| Trace solution (1000x) | g | |
|---|---|---|
| Na₂EDTA | 5 | The solution was autoclaved at 121°C for 20 minutes and stored at room temperature for up to 6 months. |
| FeSO₄ x 7 H₂O | 2 | |
| ZnSO₄ x 7 H₂O* | 0.3 | |
| MnCl₂ x 4 H₂O | 0.03 | |
| CoCl₂ x 6 H₂O | 0.2 | |
| CuSO₄ x 5 H₂O | 1.2 | |
| CuCl₂ x 2 H₂O* | 0.5 | |
| Na₂O₄W x 2H₂O | 0.3 | |
| NiCl₂ x 6 H₂O | 0.05 | |
| Na₂MoO₄ x 2 H₂O | 0.05 | |
| H₃BO₃ | 0.03 | |

| | | |
|---|---|---|
| *The growth media has higher concentrations of CuCl₂ and ZhSO₄ compared to our published media (modified from Demidenko et al., 2017). | | |

### Cultivation

Culturing was carried out in either closed vials (50 ml culture in 250 ml vials, with shaking at 200 r.p.m.) or bioreactor cultures (fed-batch or turbidostat). Two types of bioreactors were used: 1) a DASBOX^{™} (DASbox) mini bioreactor (0.5 L working volume; 200 ml culture) with two individual bioreactor units, each having automatic temperature, pH, and DO controls, a sample port for measuring OD, and a coupling to a BLUESENS^{™} (BlueSens) sensor system for simultaneous measuring off-gases (CH₄, O₂, and CO₂); or 2) a 2.7 L bench top BIOFLO (BioFlo) 110^{™} modular bioreactor (New Brunswick Scientific, Edison, NJ, USA). Cultures were also grown as batch cultures (in triplicate). In all cases we measured CH₄, O₂, and CO₂ in the headspace to determine consumption and production rates and the O₂/substrate utilization ratios using an SRI GC system. In addition, samples were taken for measuring ectoine concentrations in cell biomass by HPLC. The data were analyzed to assess yield (Y), growth rate, and O₂/substrate ratios (Table 3).

### Dry cell weight (DCW) measurement

Cultures (150 ml) from bioreactors were centrifuged to collect the biomass. After careful removal of the liquid phase, tubes of known weight with biomass were weighed (to obtain wet cell biomass weight), lyophilized overnight using a LABCONCO^{™} freeze-dry system and weighed again. The observed DCW parameters were as follows: 1L of cell culture with OD = 1 corresponds to 0.336 ± 0.025 g CDW.

FIG. 4A graphically illustrates data showing the growth of *M. alcaliphilum* 20ZR in a DASBOX^{™} (DASbox) mini bioreactor (0.5 L working volume; 200 ml culture), as batch (0-20 hours (h)) and chemostat mode (20h-90h). Steady-state was reached at 60-80h. B-C. O₂ and CH₄ consumptions and CO₂ production in steady-state for bioreactor replicate 1 (FIG. 4B) and 2 (FIG. 4C).

### HPLC protocol

Twenty mg of lyophilized biomass was re-suspended in 200 µl of water. One ml of 0.2M sodium citrate buffer (pH 2.2) was added and quickly (15 sec) sonicated to re-suspend. The mixture was allowed to sit on the bench at room temperature overnight (18 h) and then sonicated again (15 sec). After centrifugation for 10 min, the clarified lysate was filtered through 3kDa centrifugal filter units (Millipore) and the filtrate was analyzed by high performance liquid chromatography (HPLC). Ectoine concentrations were determined by using a previously published assay (He at al., 2015), i.e., an isocratic mobile phase of acetonitrile and water (70:30 v/v), flow rate of 0.5 ml/min and a detection wavelength of 210 nm. Samples (10 µl) were chromatographed using an Agilent 1100^{™} HPLC system equipped with a NUCLEOSIL NH2-HPLC^{™} column, 5 µm particle size, 25 cm × 4.6 mm (Macherey-Nagel). Pure ectoine purchased from Sigma was used as a reference (Figure 3A).

FIG. 5A illustrates a chromatogram of 1mM ectoine solution; ectoine peak area = 8497; FIG. 5B illustrates a chromatogram of 20ZR cell extract; ectoine peak area = 18066 (corresponding to 370 ug of ectoine per 20 mg of dry cells, or 1.85%).

### Results

A bench-scale New Brunswick BIOFLOW (Bioflow) 310^{™} bioreactor was used to accumulate cell biomass. A DASBOX^{™} (DASbox) mini bioreactor system was used to generate performance parameters for continuous cultures grown on methane. The parameters measured from these growth conditions include cell dry weight, CH₄ and O₂ uptake rates, glycogen content, and excreted organic acids.

The parameters for continuous culture conditions and catalyst performances are shown in Table 3, below. A maximal growth rate of 0.13 hr⁻¹ was obtained under fed-batch conditions using our standard gas mixture. The specific growth rate in the continuous culture was 0.09-0.1 hr⁻¹ (see FIG. 4) at DCW 1.15 g/L.

**Table 3. Baseline parameters for M. alcaliphilum 20Z^{R} grown in a bioreactor with methane as a source of carbon/energy.**

| Parameter | | SD |
|---|---|---|
| Gas input | 5% CH₄: 3.5% O₂: N₂ balance | |
| Gas flow | 1.6-1.7 L/h | |
| Bioreactor volume | 0.2 - 2 L | |
| Growth rate (h⁻¹) | 0.09 | 0.01 |
| Methane consumption (mmol g DCW⁻¹ h⁻¹) | 12.2 | 0.9 |
| Oxygen consumption (mmol g DCW⁻¹ h⁻¹) | 19.7 | 3.3 |
| CO₂ produced (mmol g DCW⁻¹ h⁻¹) | 4.6 | 0.9 |
| Y CO2 (%) | 0.37 | 0.05 |
| Y (biomass) | 0.46 | 0.02 |
| O₂/CH₄ | 1.54 | 0.04 |
| Ectoine (% DCW) | 1.86 | 0.07 |
| Productivity (g ectoine g⁻¹ DCW h⁻¹) | 0.0016 | 6.3 x 10⁻⁵ |

### Construction of a methanotrophic strains lacking ectR-regulator and expressing LipL1.

*Construction of 20Z^{R}ΔectR strain lacking ectR-regulator.* The strain was constructed and tested for ectoine production:
*Strain construction.* Plasmid pCM433kanT carrying approximately 800 base pairs (bp) of sequences flanking ectR gene was constructed and introduced to 20ZR strain by biparental conjugation. After mating, single-crossover, kanamycin-resistant clones were plated on rifampicin to counter-select against *E. coli.* Then, to select for Kan-sensitive double crossover clones with a deleted ectR gene, single-crossover clones were passaged on plates with 2.5% sucrose and the resulting colonies were PCR-genotyped for the absence of ectR followed by sequencing (underlined sequences, as described below).
*Results.* Amounts of ectoine in 20Z^{R}ΔectR strain are similar to the parental (WT) strain, see FIG. 6 (illustrates HPLC analysis of 20Z^{R} wild-type (batch culture)) and FIG. 7 (illustrates HPLC analysis of 20Z^{R}ΔectR strain (batch culture). However, the peak adjacent to ectoine (at 17.8min) was significantly enriched in the ΔectR strain (FIG. 7). It was suggested that the peak might be an intermediate of the ectoine degradation, thus an additional mutation, knockout of the ectoine hydrolase (*doeA*) gene, was generated.
*Construction of the 20Z^{R}ΔectRΔdoeA strain.*
*Strain construction.* The strain 20Z^{R}ΔectR was used as the parental strain. The ΔdoeA knockout was constructed the same way as for the ectR deletion. The selected clones were PCR-genotyped for the absence of the *doeA* gene followed by sequencing.
*Results.* HPLC analyses of the cell extracts showed increased level of ectoine in the 20Z^{R}ΔectRΔdoeA strain (26% more than in WT, Table 4) and no ectoine degradation intermediate (compound X) was observed, see FIG. 8 (illustrates HPLC analysis of 20Z^{R}ΔectRΔdoeA strain (batch culture)).
*Construction of the TWC#G2 and TWC#G2-2 strains expression LipL1.*

Strains for simultaneous production of lipase (as a fusion with S layer protein) and ectoine 20Z^{R}P_{SL}-L1ΔectR (TWC#G2) and 20Z^{R}P_{SL}-L1ΔectRΔdoeA (TWC#G2-2) have been made.

*Strain construction. EctR* and *doeA* genes were introduced into WT and TWC#G1.

*Results.* HPLC analysis reveals the highest levels of ectoine, 160% more than in WT 20Z^{R}, Table 4, FIG. 9A-B (illustrates HPLC analysis of HPLC analysis of 20Z::P_{SL}-L1ΔectR (FIG. 9A) and 20Z^{R}P_{SL}-L1ΔectR ΔdoeA (FIG. 9B) strains (batch cultures).

### Additional genetic strategies for improving ectoine production.

As an additional way to improve ectoine production, an isocitrate lyase/malate synthase fusion was expressed in the 20Z^{R} strain under hps promoter (Pₕₚₛ). The expression of the construct was expected to provide an additional route for oxaloacetate production, a key intermediate in ectoine biosynthesis. As expected, the level of ectoine in the strain 20Z^{R}:: Pₕₚₛ-icl-ms was increased (26% more, Table 4) compared to the wild type strain. Incorporation of the pCM132::Pₕₚₛ-icl-ms producing plasmid into strain TCW#G2-2 strain is in progress.

### Batch culture cultivation.

Growth characterization of the strain was done as described for WT. All batch cultures showed the same growth rate as WT cultures. Ectoine concentrations were estimated as described in Table 4. Each additional experiment included WT cells as a control.

**Table 4. Ectoine titer and production rate in genetically modified methanotrophic traits:**

| Averaged data (n = 2-6) for ectoine production | | | | |
|---|---|---|---|---|
| Strain genotype | Strain name | Ectoine, % of DCW | % of initial | Productivi ty (mg g⁻¹ CDW h⁻¹) |
| WT- ref^{R} | *20Z^{R}* | 1.9±0.04 | 100 | 1.6 |
| *ΔectR* | *20Z^{R} ΔectR* | 2.0±0.15 | 100 | 1.6 |
| 20Z^{R}P_{SL}-L1 ΔectR | TWC#G2 | 2.6±0.06 | 100 | 2.3 |
| 20Z^{R}ΔectRΔdo eA | TWC#G2-2 | 2.4±0.3 | 125 | 2.2 |
| 20Z^{R}P_{SL}-L1ΔectRΔdoe A | TWC#G2-3 | 3.1±0.06 | 160 | 2.8 |
| 20Z^{R}::Pₕₚₛ-ms-icl | TWC#G2-4 | 2.4±0.02 | 125 | 2.7* |

| | | | | |
|---|---|---|---|---|
| *The strain has improved growth rate (was recalculated to specific growth rate in the continuous culture as 0.12 vs 0.09 h⁻¹) | | | | |

### Cultivation in mini-bioreactor in continuous and high cell density batch modes.

Cultivation of TWC#G2-3 was performed in a DASBOX^{™} (DASbox) mini bioreactor (0.5 L working volume; 200 ml culture with two individual bioreactor units. Gas input and operational parameters were the same way as described for WT strain. Collected data are summarize in Table 5 and shown in FIG. 10A-C (FIG. 10A illustrates growth of *M. alcaliphilum* 20Z^{R}P_{SL}-L1ΔectRΔdoeA (TWC#G2-3) in DASbox mini bioreactor (0.5 L working volume; 200 ml culture), as batch (0-65h) and chemostat mode (65h-120h); Steady-state was reached at 90-100h. FIG. 10B-C illustrates O₂ and CH₄ consumptions and CO₂ production in steady-state for bioreactor replicate 1 (FIG. 10B) and 2 (FIG. 10C)).

The strain TWC#G2-3 grown in continuous culture in mini-bioreactor produce twice the amount of ectoine as WT (Table 5). The ectoine productivity was calculated as was 3.3±0.3 mg h⁻¹ g⁻¹ CDW.

**Table 5: Parameters for TWC#G2-3 grown in a bioreactor with methane as a source of carbon/energy.**

| Parameter | | SD |
|---|---|---|
| Gas input | 5% CH₄: 3.5% O₂: N₂ balance | |
| Gas flow | 1 L/h | |
| Bioreactor volume | 0.2 | |
| Growth rate (h⁻¹) | 0.09 | 0.01 |
| Methane consumption (mmol g DCW⁻¹ h⁻¹) | 8.6 | 1.2 |
| Oxygen consumption (mmol g DCW⁻¹ h⁻¹) | 11.6 | 1.6 |
| CO₂ produced (mmol g DCW⁻¹ h⁻¹) | 3.1 | 0.4 |
| Y CO₂ (%) | 0.37 | 0.45 |
| Y (biomass) | 0.59 | 0.13 |
| O₂/CH₄ | 1.35 | 0.01 |
| Ectoine (% DCW) | 3.1 | 0.07 |
| Productivity (g ectoine g⁻¹ DCW h⁻¹) | 0.0033 | 2.9 x 10⁻⁵ |

### Expression and purification of LipL1 protein.

Purification of lipase after expression in *E.coli* BL21 (DE3).

### Strain Construction.

Codon-optimized sequence of LipLlwith N-terminal His₆ tag was cloned into pET21 plasmid under T7 promoter; the construct was introduced into *E.coli* BL21(DE3) strain.

### Expression and purification.

Cells were grown in 300 ml of LB with ampicillin (100ug/ml), at which point lipase production was induced by addition of IPTG (0.5mM final) at OD600=0.5 and continued for 7h at 37°C. Cells were collected by centrifugation. For purification, cells were lysed by French Press (purifications 1 and 2) or by sonication in the presence of 0.5% Triton X-100 (purification 3), clarified lysate was loaded to Talon resin (Clontech) for one-step purification by metal affinity chromatography. After washing of the resin and elution of lipase with 200 mM imidazole, lipase prep was dialyzed against 20 mM tris-HCl (pH 8.0) and 100 mM NaCl buffer followed by addition of glycerol to 50% w/w and stored at -20°C.

### Activity assay.

Activity of the isolated lipase has been confirmed on Rhodamine B plates and by p-nitrophenoldecanoate assay. One unit was defined as the amount of enzyme that released 1 µmol 4-nitrophenol.

### Purity validation.

Purity of the purified lipase was checked by electrophoresis on SDS-PAAG (12% mini-Protean TGX^{™} gels, Bio-Rad) according to manufacturer's protocol. Gels were analyzed and quantified with IMAGELAB (ImageLab) 4.1^{™} software (Bio-Rad) and are shown below (Fig. 7); in all 3 cases only the protein band corresponding to LipL1 is visible, no other (contaminating) proteins are present.

Three sets of LipL1 expression and purification were performed. In total, about 12mg of pure L1 lipase were isolated.

**Table 6. Summary of LipL1 protein preparation**

| Preparation | Volume ml | Protein mg | Specific activity* U/mg |
|---|---|---|---|
| P# 1 | 1.5 | 3 | 630 |
| P# 2 | 2 | 7 | 400 |
| P# 3 | 2 | 2 | 1200 |
| Total | 5.5 | 12 | 589 |

| | | | |
|---|---|---|---|
| * 1 unit (U) is the amount of enzyme that catalyses the reaction of 1 umol of substrate per minute. | | | |

### Lipase production in methanotrophic strain TWC#G1, TWC#G2 and TWC#2-G2:

Different constructs have been made for production of lipase from plasmids (under different promoters) and from genomic DNA as a fusion with S-layer protein. All of the strains have been shown (qualitatively) to produce active lipase by both Rhodamine B and p-nitrophenoldecanoate assays. The specific activity of methanotrophic lipase in TWC#G2-3 is 1.2 U g⁻¹ CDW. FIG. 11 illustrates LipL1 preparations.

### Construction of strains TWC#1, TWC#2 and TWC#2-2:

The coding sequence for LipL lipase was introduced into the 20Z genome as C-terminal fusion with S-layer protein of 20Z^{R}. A HRV3C protease recognition site was placed in frame between the S-layer protein and lipase sequences to allow protease cleavage of the fusion polypeptide and release of the free lipase. The codon-optimized LipL lipase sequence (synthesized at GENSCRIPT^{™} (GenScript)) with the HRV site was introduced by PCR. Plasmid pCM433kanT carrying approximately 800 base pairs (bp) of sequences flanking the fusion site of S-layer protein was constructed and introduced to the 20ZR strain by biparental conjugation. After mating, single-crossover kanamycin-resistant clones were plated on rifampicin to counter-select against *E. coli.* Then, to select for Kan-sensitive double crossover clones with inserted lipase gene, single-crossover clones were passaged on plates with 2.5% sucrose and the resulting colonies were PCR-genotyped for the presence of lipase followed by sequencing.
Genomic region of ectR (MALCv4 3251) with upstream and downstream flanks. The genetic region deleted in ΔectR strain (ectR gene) is underlined) (SEQ ID NO:1): 
gaaccgctttgaacggcccaaccttcaccagcatttcggtttcatgttgatctgcaa
aatggcgtgtcttatcaaacatcacagcactgccaatttgagtgtccagtttttttg
ccagcccctcaaacagagcccatgaggccttattattcggagtaatggtcgtctcga
ttcggttaatatcctgattgaccggccgcgccagtatggctttcagcatccgcgtgg
caagcccttggccacggctttttcgccgacagccacctgccagacaaacagcgtat
ccggacgttgcggaatacgataacccgagacaaaaccaaccaactcatcgccaattt
tggccgccaccgccgtttcagaaaaatggctgctctgcagcaaattgcagtacatcg
aattgggatccaggggcgggcatttgctaatcagccgatgcacctgcgctccgactt
cggcagtaggctggctaagtgtaataatcggcaaggcagttttatcaggcaacatat
aaataactctattatttagatttctgtgcaattaactcggctttaactgaataagcc
gggctcgaatttgatttttttatggccatcagcacgaatattctggcttcattgaaaa
acataatatatagtacactaaataatttaaatgtccaggccgcgtacttttgcctta
gattaattagatgtcatatcaaattatgcctttcgaacttcaaatttcggtagcgcc
ctaggatgcgccgggcggagcaccgaattttgttcttcgagggtatacaataatggc
tttgtaacgcgacggcctctatttcaatgattggtgatcaatgatgcaaacccaca
accgcacgcccctcattcgctggatacgctcgacttgaatccggttgaaaggaaca
tttgctgaatcaaattgaagaagtactggtcgcgttacgtagagtgattcgcgccac
cgatttacactcaaaatatctggcaaaaaccactagcctgaccgcaccgcagattct
tttgttgcagacactgcgcgccaaaggtcaactgaccattggtgagctagctcagga
catgagtctcagccaagcgactgtgacaacaattctggatcgcctggaaaaacgtca
attggtgttccggcagcgctcccagactgataaacgaaaagtccatgtctatatgac
ggaggcggccacggaaatgctaataaacgcccctatccctttgcaggatcgctttac
gcgagaattcagtaaactacaggaatgggaacaattgatgattattgcatcactgca
acgtgtcgctcagatgatggacgcgcagaacatccctgtcgctaaagaagcgtttga
ttttccggtttaagctctaataattcagctcagctgcaacccgcatcacgcttttc
ccaagctccagcttgggaaaaacaccccggaagctccagcttccagaaaccgagata
acctccgcacattctcaatcaaccccgactcgctcgttcaatctttttcctgattag
caagatgcttcaacttgctgaagccaattgtccgagcagtggccagtcgtagccact
tctgcgggacgggttatttaacccatccccaacgtttcggtttgccctaaacatttc
ggctgacttcggccaaagtcaaaacgtttaggacggggctgcaaaccccgtcctgct
aaggatatgctggttttcgggctttagctgaagaaacttgctaatcaggatcttttt
tgattgtcgggaagctagagcttcctgaatagattacccaagccggatcgctcgccg
ctatacacaaatatcggtaaacttgctaaacagaggtcgccgtatacttggaagcgg
tgctgtttgataaatctgcggatattggacatcagtggcttcgaagtcggaacgtt
gggcgataaaaaatggtatcgaggcctcattggttaagattgcaaaagggtaatttt
tgacttatgtataacgatgaggtaactattcagtcccccggcaattatcgttcccac
gctctgcgtgggaatgcctgagtaccgctccagcggtacgagacgctagagcgtctc
ggtcttcattcccacgccggagcgtgagaacgataggcggtgtgaataattacacga
tgagagctggagcttgggtaacagcacaaaggcatatttagcctagttgc
Genomic region of *doeA* (MALCv4 3246) with upstream and downstream flanks. The genetic region deleted in ΔdoeA strain (*doeA* gene) is underlined (SEQ ID NO:2): 
gtagcaagccttgcgtagagattattgccgggtgtaaaggcgtctatgctttcgagg
tgttcgatcaaaacatggccggcgagattcacaattacgatcggaaatcctggggc
aaattcgccgcttcagagcgcatattgtttccaaagatattaatgccaataccatta
ctcattatttgtctgccagtctgaaaacaattcgccgcatccgcgatgccttgcaag
aaatctatccggatgccgaaatcaatcagcaaaaagtttcgattgtttccgccatcg
gcagtgacatgaaaattcccggtattctggccaaaactgtctcggcgctggcagaga
agcagatcagcgtgcttgcaatgcaccagtcaatgcgtcaagtcgatatgcagtttg
tgattgatgaagatgcctacactgatgcgatgaaaagtttgcattgtcatctggtgg
aggtccatgatcacggcattgcaatatgcctcgcgtcctgattgtactgatgtttct
taccccctaaatacgggaaatttctcaaactgggaattgctgccaaagaaatgaaaa
tgccttgcgtcttcagtcttgcgctgaacgacaaggaagcgcataaggcttaaagcg
tttaccactcgaccgctgaagcggtagcggattaaggcgagtcgcaagtcaattttc
 
gtccaaggttaggttgttcatggcaagtcagtcgagcaatgaatgacttaaccgtct
atttttcaataaactgaagatgtacgggtaagccctgcgtaagttgggaatggccga
tgatcgagggctatctgttgtcgcgaagtctttaatcaaaaaaatgggtttaatatt
caatgattaccgagaatgccgcacagtccgaacaaagtgaagattttatcaatcac
gtaacggtagtaagccgaaaataattccgcgcgtagacccggtagtttatgcgcaaa
cagctaatccaggtctcattgcagaggacttgcaagcacgttatgagcaacaaggtt
ttcttgttattgataatgttttaatgagagggaggtcgactgtttcaagcaagagc
tcaaacgcttgaacgacgatgaaaagataaaagcctcggcggaagcgataactgaat
tatccagcgacgaactccgttcactatttaaaattcatgaagtcagtccggtttta
aaaggttagctgccgataatcgattagcggactggctcaacatcttttgaacgacc
gggtttatattcatcagtcgcgcttaaactataagccgggtttcgcggcaaggaat
tttactggcattcggactttgaaacttggcatgtagaagacggtatgcctagaatgc
gtgcgctcagcatgtccattattcttaccgaaaacgatcagcataacgggcctttga
tgttggttcccggatcgcataaaaaatttgtcgtttgcgaagaggaaacgccggaaa
atcattattcggtctcgttgaaaaagcaggagtacggcatacccagcgatgaatgct
tggctagcttggttgccgatggcggcatcgtatcggccaatggaaaacccggcagtg
tcttgatttcgacagtaatgtcatgcacggttcgaatagtaatatcactccatggc
ctcgctcgaatctctttttcgtctataacgcgatcaataatcgagtaacatggccgt
tttgcggtttattgccgcgtcctgaatatctttgcagtcgcaagaatatacgagtta
tcgaaccgcggcctttatcgcggccgccgatcaattgatatatgcttagaatgtta
ataatgttgatcgtgctggcgccctgttccgtgttgggcgagagcgtcaacgatgaa
gcagaggttcaagagcgcttagatgcggttgaatctttggataagcctttatatagt
ccgttcatcgagcgctatatgctggatgaactcaaacaattgcgtatggacatggca
gcgcagaggaatgagctgattcagcaaattgtggatagagagcttagctcggtcgat
agaggcgttacttacgccactaatactgtcacatatttttctacttgattgccggt
gccagtaccattttggtgttgctgggttggacctcgctcagagatatcaaagagcgt
gtgcagtccatggcggataagaaagtatcgaaactggtccatgaatacgaagagcgc
ttggcaattgtcgaacaacaactcaacaaggaagcacaattgattgagaaaatcggc
gaggatatcgggcggacgcaagatgtgcaatctctctggcttagagcaggtcaagca
ggcagcttggccaataaaatcgccatctacgatcaaattttaaaattgcgtcccgag
gattgcgaagcattgacttataaggccgatgcggtactcgatatgggcgagccgcag
tgggccgtcaatttatgtcagcaagcgttgaaaatcgaccctgaaaacggccatgct
ttttaccaattggcttgtgcgtataccgcattggatcaatatgaagaggccgttaac
tgtttatccgaagccttggcgcgtaccgaggattatcgcgataagtttgccgatgac
cccgcgctgcaagcgttaaaaggttttgagccgt
*LipL* sequence (His6 tag is underlined) (SEQ ID NO:3): 
ATGGGTCATCATCATCATCATCATCTGGAAGTCCTGTTTCAAGGCCCGATGGCCTCG
CCGCGTGCGAACGATGCGCCGATTGTGCTGTTACATGGTTTACGGCTGGGGCCGG
GAAGAAATGCTGGGTTTCAAATACTGGGGCGGCGTCCGCGGCGATATCGAACAATGG
TTGAATGATAATGGCTATCGCACCTATACCTTGGCCGTCGGCCCGTTGTCGAGCAAT
TGGGATCGCGCGTGCGAAGCGTATGCCCAATTGGTCGGCGGCACCGTCGATTATGGT
GCCGCGCATGCCGCGAATGATGGCCATGCCCGCTTTGGCCGCACCTATCCGGGCTTG
TTGCCGGAATTGAAACGCGGCGGCCGTGTCCATATCATTGCCCATAGCCAAGGCGGC
CAAACGGCCCGTATGTTGGTCTCGTTGTTGGAAAATGGCAGCCAAGAAGAACGCGAA
TATGCCAAAGAACATAATGTCTCGTTGAGCCCGTTGTTTGAAGGCGGCCATCGCTTC
GTCTTGTCGGTCACCACCATCGCCACCCCGCATGATGGCACCACCTTGGTCAATATG
GTCGATTTTACCGATCGCTTTTTCGATTTGCAAAAAGCCGTCTTGGAAGCCGCCGCA
GTCGCGTCGAATGCCCCGTACACCAGCGAAATTTATGATTTCAAATTGGATCAATGG
GGCTTGCGTCGCGAACCGGGCGAATCGTTTGATCATTATTTCGAACGCTTGAAACGC
TCGCCGGTCTGGACCAGCACGGATACGGCCCGCTATGATTTGAGCGTCCCGGGCGCC
GAAACCTTGAATCGCTGGGTCAAAGCGTCGCCGAATACCTATTATTTGTCGTTCAGC
ACCGAACGCACCTATCGTGGCGCCTTGACCGGCAATTATTATCCGGAATTGGGCATG
AATGCGTTTTCGGCCATCGTCTGCGCGCCGTTCTTGGGCAGCTATCGCAATGCGGCC
TTGGGCATTGATTCGCATTGGTTGGGCAATGATGGCATCGTCAATACCATTTCGATG
AATGGCCCGAAACGCGGCAGCAATGATCGCATCGTCCCGTATGATGGCACCTTGAAG
AAAGGCGTCTGGAATGATATGGGCACCTATAAAGTCGATCATTTGGAAGTCATTGGC
 
GTCGATCCGAATCCGTCGTTCAACATTCGTGCGTTTTATCTGCGTTTAGCGGAACAA
CTGGCGTCCCTGCGTCCGTGA
Sequence of the S-layer protein (MALCv4 0971)-Lipase fusion incorporated into *Methylomicrobium alcaliphilum* 20Z^{R} chromosome. HRV protease site is single underlined (i.e., is ctggaagtcctgtttcaaggcccg), and the lipase sequence is shown bold (SEQ ID NO:4):

atggcaacactctcagtggatatcgctcaatcctatatggagaccttacggtcctat
gggcttgagtttaacataagacaaaccaacaacctgaccaatcggataattaaccgc
ttggaaaatcgcggccacacacctgagcaagtagcagactggcttatgagcagggtt
gcggttaaacagcaattgagaaaactggttaaacaaggcgaattggccgagtttgat
ctggatggcaatggccgcctcaatcgatctgaattgctaaatgcaatgtctgctctg
tccgagacagcggtcgaagaagcgccggtcgaagatcccacgactcccaagcctccg
gccgatcctagcatcacgaccttaacgcttactgagattccgactcagcgtacggct
acgttaaaatggaacaatgtcgatgccgatttggccatcgatttcatgcaagacgta
ctgaaactagatctaaatcgactaggctggatggaagacggtcaattgaccgtcaac
atcgacaatatcgcgatcagcgattcggacagtaattctgatatcaacatcggtatg
gtcgatggcgaagaattttgttcagcgtaaatacgccagtggcgctgtataccaat
attatatttgatttgaagcaaaacgatgatgtcattcaaaccggcatcgtgctaacg
ccgaccgaaaacaacggcggttcgtttgaaaacggcattacctccgatgccgacaac
cacatcatcgccggtcgtcctgaattgctgcacggcgcctacatcgacggcggcggg
ggctacaacacgttggaagtcgacatgaaaggcttctttgcgcagccgttccaactg
ttgaacatccaagagatccacgtacaaaacctcccgaatgtctacagtttcgatcaa
acaattttcagcgacaccgaaggcgactatttgctaattttccaattcctacgaat
ttggatggcgatgatagcattcttgacttgagccggccactagcctagaaagactg
gtcattaacgaagcacgctttcccggtagcgcaaatgccttaggcgacctctacctg
gtcggtatcaaagccgatgcggtcgcccgtctagaaggcaacttcaccgaagacgta
aacttgttctatggtcgcggcttgggtaatgcgatcaacctggaatttgccaatgtc
acgatgagtgatagtgagggggggggtgaattggtgctgggtcataatgccggtacc
gtgaatctgctttccgaaggtcgtctgaacgtcttggaatctgttgatttcggtagt
ttcctgcgcgaactcaccatcaccggtaccggagagttggttattgatgacgccctc
gcattcgcctttggcgaagtacatatcgatgcctctgctaataccggtggtattcgc
ctcaaggtcgacagcgttgcagacggtagcagcctatccaatgaaatcggcttttct
gcggttcttgacgaagtcaccatcaaaggctcacaaggtcgtgacgttatcgagatc
tccggcactgctgcaggcgtattgcttgacatcgacaccggtgctggccgcgacacc
gttttcttgaccgacgatactttgagtgctggcgctggctcagtgatcaccggtgat
aatttgacagttgtggtgacagccaccgccgatctgcgtaaggccgatgttgttggc
gttgaccgctttgtactgaatgcaggtcccgcagccgctggcaacctggttttgact
cagacccaagtagaagccatggatgccggtgtgttcaccgcagcccataatactatc
gcagttctgtcagtcgaaatcaccgaagcgggtacggttctgtcagatctgatcgat
ctttcggcactgagcagtgatgttaagctggccttcaatgttgttaaaggtgcaagc
cttgaaatgaccgctgaagaactgcataagtatgtagcttttgaaggcatcgatgca
actatggctggtcacctggtgatcaccggtgcgggtctgggctttgatcctgaagat
caatctgactacgatactggtggtacgattgccaattacggtcttacaccagaccaa
aatatctccattattcgtgatccaaacggttttgagcgcccggcgcccgataccaac
actgacatcctgaccattgataccacaggtggaatcaccatcggtgccaatgcactg
tcagatgacgatgccttctcaaccaatgcaaccaccttgatcatcgaaggtgcaggt
gatattacctttaatgcaccgttggaaatgttattagataactacaccatcgatttt
tccggcctgaccggcaatctcaatggcctgaccattctcgatttccagaacatcacg
gatggaaacgatccgagcgactggggccagattatcggtaaccccgatgttaatacc
cgtatcaacgtggtcattgaggacggtaaggaagttggtgatgacagtttaggcaat
gccaatggcggcctcaagtcttccggtgtcgaaacctacgtagttctggaactgtc
ggcgaaacctacaccttcaatgtttgtgataccacgcaaggccttgaagtcctcggt
ttccgtggtctgggtgatgtcacgttcaaccagatcaactggggcaccaacctgctg

ctggaaggcgatggctttgaaactttggtgatattccgaaggcttttgccaacccg
aaccaatccaacatcggcagcatcgaagccaactacttcttcgatggtgctactgta
gatgttgccatcaacaaccagggtcaagctctgggcaccacctctacagggcagcg
cgtcccttagtggtagaaagcatcgtggtaaacggtgctgagaccgtaaacctggca
atcgaagacggcagcgcctggatcaaatctgttgatggctctgttctcgaagatctg
accgtcaccagtgatttccacgttacactctcgctgatcgctgccaacagcgacctc
gaatctatcgatggatctggtgtcgtaggtgtcatggctctggagattagtgatgtt
gatggcgccggggatcctactgccctgaccgtagacctctcttctacagaactgagc
ggcatcgaccagatcgatctgggcccattggctgatctcactctgaacattgatcag
attgaggacatcggcacagcaaacatcgcctttaccggtacggctgcacaagctcaa
aatgatccagcgacgctgaacattggtcagtttgctgaacaagagtttgatattacg
acagtcggtctggaagccggtgttgaactgggtactgttacctttgttgcgaatgca
ggtgaaatcactatgcatccagacaccaacctgtccggtgcaactgcaatcgtgatt
ccagaaggcacaaccgtgaatatgacggctgcgcagtatgagcagatcgtagatggt
ggaaacggtagcagcttctcaggcctcggtgtgttgaacatcaccgacctgctgggt
gagcctgaactggatacaaatggtgatccgatcccaggagcctttgcttccgacatc
aatctgtccggtgtacctgtcgaaatgatgggcagcatcagcctggcagcgggtgtc
gatagcatgcgcctgacgggtaatatcggtatcgctcagtttgaagaaaggaaatt
gctgatcctgataatacagacttcgtatcggttcagacttcgcataattttcatgaa
ctgaccggcgaaacgcaggcttcagctttgtgctggccgaagatcagaacctgatc
ttcaccacagaagcgcaggcccataaccgtgtggttgaaggcgatggctctaaagtc
acgctggcgtttgctgtgctgctggactctcaggtcaacttcaatacgctgggcggc
actcttgctgttgatggtctgaacctggcctactacagcgatttgaccgatcttgaa
gtgcttcaggccttggtagctggtactaacgttgagcagatcctgggtaatctggat
gaaaatacagttgttcagatttctgagtttattgctggcgctaactttgctaatccg
actttccgcgatgtggaagtgttggaaggtgtaactgttgccggcggtctggtcttc
gagaacctgaacgatgagttaccggatagcctcaagctgactgaactgagtctcagc
ctgttgggtgatgcgactatcacaggtacggttgatattagtggtgcaccactgctg
gatcagggcttcgaaaccctgaccatcaactcgctgggtgatgatcccaacaccatc
aataatgtcgtagcgacaggcaacgatctgatcgatgtcgtgatcaatgctgaacag
gatctgtcagttcaaaccatcaccttgagctttgttccaaaaacacctggtcaaaca
tcagacgcaaccttgacagtcaatggtgatgcagatgtaaccatcaagacactggat
agcactgatcctgatatcaacgtagttaacattgataacaacctgactggcggtgcc
acactcaccttcacaggtggttcagctgcattcgagggtgatgatacagatagcctg
atccttaccggtgcaggtaacactgtatttgatactgaaggtgcaagtacgggtggt
atcgactccgatagcctgtcactgatcgatgcttctgagcacaccggtgatctggac
ctgggtcgtatcatcagtgttgacgaagctaacttcagcctgctgacctccgccggt
aatgcctcagctacgctgcaagccactatgaatgatcaaggctttaatgccgcggtc
ctggcttacaacgccgctctggcagcggatcctgttgttccgggaaacgttactgca
gcgctaactgctctaactactgctgcaggtcttcttggctttgtagatgcaaatgaa
gatccgctggccttcactcaggctaacgcggctgatctgatcgcagagttccgtcct
gaatggaactttgagctgggtgctaacaccgagctaaccatcgatggagatgacatc
gttggagctgactttgttgccggtgccctggtgatatccggtggcaagttgatcatc
gaaggtgaagtcgatctgcgtgatctggctgtactggacatctctgatgtcgagatt
gagctggccgcaggtgcacgtatcctgatgactgacgagcagttcgacgcgctggac
aacgtcaccttctctggcccaggtcagacgctggaagttgatgatgcgctgctggct
gagctttctatcgtcaacgatatcactgatattcgcggtgtcactgagattcagttg
gaagaaggcctggttgaagacatcaccatgacagctgagcaggcgcgtatcgcgact
gtagtcgatgccgacggtaaccctgtcctggttgacttcgatgttgatccaactgtg
atcgatgctgcgggtgaccctgttgaagccggagacttccgtacccttacaggttct
gttgttaccgtcgaagtaacaggtaacgacgatctgaccgatctggctggcctgaat
cgcattgaaatcgtcagtgatgatctggttgatctgaaagtggcactggatcttgat
ggtgctaacaaccaggttgccgcgttgcaaactgcagcgggtggagcgtttgacggc
acattcttcgatgtgttgagcaacttcactgttgaagcaagctttgaggtgctgagc
cagtttgaccctgaaaccacgttgttcgttgccaatccgatcgtggaggatgtcaac
ttcgacatcgtacgtgatgtgaatggtgatgtgacttcagtcagcgtcagcggtggc
tcttcccttggtttcgcccagagcgatgccggcttccaggagttgttggaagccggt
caggtaactgaggtggtgttcgagaatgttggttcactcaacagcatccttgttagt

ggcaacttcgtcggtagctacgatgccggtggtattttctacgagagcacctttgag
ttcggcgcaaatgctggttcggttgctgaagggtgggtacagatggcaacatcttc
accattgctgaattcacagccggtgcagcagccagtgatatccttgatttcactgcc
atgcctgttgataacacgaacactgctccagccactgggcatgagttcatcgcggta
ggcactgaagctagcattggtgacgatgccaccatcattgtcttcacggcgggtgtt
gcggccgacgcagcaaccatcgtgacacagtttgctgatggtgcgggagattccgt
tcagcagatgctactgcacgtaacgctgactttgctattgatagccagttgatcttc
ctgattgacgatggcgctggtaataccggtgtctggtattgggatgatacagttggt
gctgttggcgatggtattgtcgatgctgatgagctttcgcagattgcccagttgact
ggagtcgtcactgccgagctgacggttgataacttcgtcctcgctctggaagtcctg
tttcaaggcccgatggcctcgccgcgtgcgaacgatgcgccgattgtgctgttacat
ggttttacgggctggggccgggaagaaatgctgggtttcaaatactgggcggcgtc
cgcggcgatatcgaacaatggttgaatgataatggctatcgcacctataccttggcc
gtcggcccgttgtcgagcaattgggatcgcgcgtgcgaagcgtatgcccaattggtc
ggcggcaccgtcgattatggtgccgcgcatgccgcgaatgatggccatgcccgcttt
ggccgcacctatccgggcttgttgccggaattgaaacgcggcggccgtgtccatatc
attgcccatagccaaggcggccaaacggcccgtatgttggtctcgttgttggaaaat
ggcagccaagaagaacgcgaatatgccaaagaacataatgtctcgttgagcccgttg
tttgaaggcggccatcgcttcgtcttgtcggtcaccaccatcgccaccccgcatgat
ggcaccaccttggtcaatatggtcgattttaccgatcgcttttttcgatttgcaaaa
gccgtcttggaagccgccgcagtcgcgtcgaatgccccgtacaccagcgaaatttat
gatttcaaattggatcaatggggcttgcgtcgcgaaccgggcgaatcgtttgatcat
tatttcgaacgcttgaaacgctcgccggtctggaccagcacggatacggcccgctat
gatttgagcgtcccgggcgccgaaaccttgaatcgctgggtcaaagcgtcgccgaat
acctattatttgtcgttcagcaccgaacgcacctatcgtggcgccttgaccggcaat
tattatccggaattgggcatgaatgcgttttcggccatcgtctgcgcgccgttcttg
ggcagctatcgcaatgcggccttgggcattgattcgcattggttgggcaatgatggc
atcgtcaataccatttcgatgaatggcccgaaacgcggcagcaatgatcgcatcgtc
ccgtatgatggcaccttgaagaaaggcgtctggaatgatatgggcacctataaagtc
gatcatttggaagtcattggcgtcgatccgaatccgtcgttcaacattcgtgcgttt
tatctgcgtttagcggaacaactggcgtccctgcgtccgtga

### Example 3: Exemplary Methods and Compositions

This example provides exemplary methods for making compositions and bacterial cells as provided herein, and practicing methods as provided herein.

### Producing lipase outside of the cell as N-terminal fusion to S-layer protein.

Previous attempts to generate C-terminal fusion of lipase to S-layer resulted in no lipase activity and no S-layer in mutant cells. Upon thorough theoretical analysis, it was hypothesized that fusion of lipase to the N-terminus of S-layer proteins should solve the problem and be sufficient to ensure transporting of the fusion to outside of the cell.

Two genetic constructs comprising an exemplary recombinant polypeptide: (i) Green Florescent Protein (GFP) and (ii) L1 lipase fused to N-terminus of Slayer protein were generated and introduced into 20Z chromosome (in a 20ZR-L1-SL strain).

GFP-S layer fusion synthesizes active GFP which is distributed throughout whole cell volume which is in agreement with its putative outside localization, as illustrated in FIG. 12. Moreover, those cells excrete GFP-containing crystalline-like material which accumulates in extracellular media.

The strain TWC#11 (20Z^{R}:: SL_{Nter}-LipL1, N-terminal fusion) yielded 133 U/ g DCW of lipase, the majority of which was localized outside of the cell. The lipase is fused with S-layer and expected to co-purify with S-layers. Initial tests with the strain TWC#11 indicate SLNter-LipL1 fusion is loosely attached to the cell wall (Table 1). We tested a previously published protocol (see Shchukin V.N et al., 2011, Mikrobiologiya. 80: 595-605) for separating S-layers. Alternative protocols for S-layer separation can also be applied as described e.g., in Hasting and Brinton (1979); Sara, M, et al, J Bacteriol. 1998 Aug; 180(16): 4146-4153; or, Sleytr, UB, et al, FEMS Microbiol Rev. 2014 Sep; 38(5): 823-864.

Finally, we tested the applicability of inteins for protein expression. Two methanotrophic strains were made, and two genetic constructs containing self-cleavable intein were inserted between lipase and S-layer were made (as illustrated in FIG. 13, including:
(i) Mxe GyrA intein, which is activated by addition of thiol reagents like DTT, beta-ME, etc.; and,
(ii) Ssp DnaB mini-intein, which is activated by pH shift to 6.0-7.0.

The strains of *M. alcaliphilum* 20Z^{R} with Ssp DnaB mini-intein and Mxe GyrA intein were obtained. The strain Ssp DnaB intein showed lipase activity; however, the activity per dry cell weight was about 50-60% compared to N-terminal S-layer-lipase fusion (with no intein). About 70% of that activity is localized inside the cells in the soluble fraction, suggesting that the intein cuts inside of cells. The difference in extracellular lipase localization is illustrated as the Rhodamine B assay in FIG. 14, where the strong magenta color indicates lipase activity.

**Table M4-1. Lipase activity as determine by p-nitrophenol assay.**

| | 20ZR::SLNter - LipL1 | 20ZR::SLNₜₑᵣ-LipL1-ssp intein | 20ZR::SL_{Nter}-LipL1-ssp intein |
|---|---|---|---|
| | | *Mutant 1* | *Mutant 2* |
| whole cells (U/gDCW)* | 34.6 | 9.7 | 7.5 |
| supernatant | 8.3 | NT | NT |
| cytosol/envelops (U/mg protein) | 14.9 | 9.2 | 12.0 |
| cell debris (U/mg protein) | 0.6 | 1.4 | 4.5 |

| | | | |
|---|---|---|---|
| *Whole cell assay-only cell-surface enzyme are active. SD ≤ 5%; NT, not tested. | | | |

These results lead us to conclude that the majority of lipase-Ssp DnaB mini-intein-S-layer protein fusions are self-cleaved inside the cell with only a minor fraction exported to the outer cell surface.

Several mutants of 20Z^{R} strain harboring LipL gene fused to S-layer protein via Mxe GyrA intein has been constructed. The genotyping of the strains was carried out, and we show that all mutants harbor the LipL (see FIG. 15, left), and the gene locates in correct orientation LipL-MxeGyrA-S-layer (see FIG. 15, right).

Since LipL- Mxe GyrA intein requires high concentrations of thiol reagents for cleavage, the chance of intracytoplasmic self-cleavage of that construct are minimal. An alternative approach for lipase expression includes the addition of a C-terminus to the lipase gene.

### Expression of GFP protein fused to C-term of S-layer protein

We found that S-layer proteins are excreted via Type I secretion system. The benefits of this systems are as follows: typically proteins are produced and folded in cytosol; Type I secretion systems recognizes a specific tag at the C-term of a protein, upon recognition the protein is translocated from cytosol to extracellular environment. If efficient the system would enable direct production of the targeted proteins in cell culture. Several GFP construct fused with 900bp, 300bp, 108 bp and 36 bp of C-term of S-layer protein were made. The construct is expected to carry C-term recognition domain, which is used by Type I secretion system for the protein export outside of cells. Out of five constructs, three were obtained (900bp, 300bp, and 36 bp). The images of cells harboring GFP-fused proteins are shown in FIG. 16. Relative fluorescence of the supernatants was measured using a fluorimeter and background fluorescence in wild type cells compared with the construct strains, see Table 3, below. The GFP construct with 36 bp of S-layer C-term display highest fluorescens in supernatant, and has similar to other construct GFP per cell, indicating that the construct is efficiently exported from cells.

**Table 3. Relative fluorescent units for GFP-fusions.**

| C-term fusion | Supernatant (RFU) | RFU per cell (GFP/µm²) |
|---|---|---|
| | | |
| 300 AA (900 bp) | 1200 | 105.43±20.82 |
| 100 AA (300bp) | 3025 | 103.15±38.68 |
| 12 AA (36 bp)* | 28899* | 110.31±23.15 |
| WT | 1544 | none |

Exemplary recombinant polypeptide sequences, as discussed above, are:
(noting that all the exemplary recombinant polypeptides, below, have C-terminal S layer protein domains)

Slayer_{Nterm}-L1lip fusion, sequence
(the following 3 domains are linked to constitute the complete recombinant protein, however, the individual domains are separated from each other, below, so that the sequence of each domain can be identified) (SEQ ID NO:5):
*upstream sequence, non-coding* 
tttaacattctaaatggcgcaagtccgtataaatttaccacgaatcgggtttaatcgcagagcggcgaggcatcgttttacaa
cccgcccggcaaagaaagtgttgtgggggcgacgcctacgtcgcgataaatcgaagtcgtgtcgctaatcgacaaaaca
aacgtactcgaagcccctacggcacgaagcccggcagcgtagcggaattcgggaatggcctggctccgaactccccgg
attgcgccatgctccatccgggctgcgctgtttagactcgccgaaagcggtcggtagcgatttatcagcgagaaaccttgtt
aactttatgcgtatgggcgacaaccccgtccggcataagggcggcaaagaaagtgttgtgggagcgacgcctacgtcg
cgataaatcgaagtcgtgtcgctaatcgacaaaacaaacggactcgaagcccctacggcacgaagcccggcagcgtagc
ggaattcgggaatggcctggctccgaactccccggattgcgccatgctccatccgggctacgttgtttagactcgccgaaa
gcggtcgctagcgatttatcagcgagaatctttgttagctttatgcgtatgggcgacaaccccgtccggcataagggcaac
aaaaagttcagctatggaatcgatatatcgataaataaacaatgaaaaacattttaattaaatcaaatatataaaacttaaatta
cactaaaatacttcaatcatagtaatagaaagccaaattttaagcattatttcacccaaaataagggcggtccctagaaaaatt
attaaaaactctctatactcaagcaccgtaagctatcaactcagtccagctctttacttagaaaggctgatcaaggtatagtgc
atacaaaattcagtgcgtatcaaaacgtgtctagagttctttctaacaaaaagcgaaaacctcaattggagatttaac
*L1 lipase* 
atggcctcgccgcgtgcgaacgatgcgccgattgtgctgttacatggttttacgggctggggccgggaagaaatgctgggt
ttcaaatactggggcggcgtccgcggcgatatcgaacaatggttgaatgataatggctatcgcacctataccttggccgtcg
 
gcccgttgtcgagcaattgggatcgcgcgtgcgaagcgtatgcccaattggtcggcggcaccgtcgattatggtgccgcg
catgccgcgaatgatggccatgcccgctttggccgcacctatccgggcttgttgccggaattgaaacgcggcggccgtgtc
catatcattgcccatagccaaggcggccaaacggcccgtatgttggtctcgttgttggaaaatggcagccaagaagaacgc
gaatatgccaaagaacataatgtctcgttgagcccgttgtttgaaggcggccatcgcttcgtcttgtcggtcaccaccatcgc
caccccgcatgatggcaccaccttggtcaatatggtcgattttaccgatcgctttttcgatttgcaaaaagccgtcttggaagc
cgccgcagtcgcgtcgaatgccccgtacaccagcgaaatttatgatttcaaattggatcaatggggcttgcgtcgcgaacc
gggcgaatcgtttgatcattatttcgaacgcttgaaacgctcgccggtctggaccagcacggatacggcccgctatgatttg
agcgtcccgggcgccgaaaccttgaatcgctgggtcaaagcgtcgccgaatacctattatttgtcgttcagcaccgaacgc
acctatcgtggcgccttgaccggcaattattatccggaattgggcatgaatgcgttttcggccatcgtctgcgcgccgttcttg
ggcagctatcgcaatgcggccttgggcattgattcgcattggttgggcaatgatggcatcgtcaataccatttcgatgaatgg
cccgaaacgcggcagcaatgatcgcatcgtcccgtatgatggcaccttgaagaaaggcgtctggaatgatatgggcacct
ataaagtcgatcatttggaagtcattggcgtcgatccgaatccgtcgttcaacattcgtgcgttttatctgcgtttagcggaaca
actggcgtccctgcgtccg
S *layer protein (in frame with L1 lip)*

gcaacactctcagtggatatcgctcaatcctatatggagaccttacggtcctatgggcttgagtttaacataagacaaaccaa
caacctgaccaatcggataattaaccgcttggaaaatcgcggccacacacctgagcaagtagcagactggcttatgagca
gggttgcggttaaacagcaattgagaaaactggttaaacaaggcgaattggccgagtttgatctggatggcaatggccgcc
tcaatcgatctgaattgctaaatgcaatgtctgctctgtccgagacagcggtcgaagaagcgccggtcgaagatcccacga
ctcccaagcctccggccgatcctagcatcacgaccttaacgcttactgagattccgactcagcgtacggctacgttaaaatg
gaacaatgtcgatgccgatttggccatcgatttcatgcaagacgtactgaaactagatctaaatcgactaggctggatggaa
gacggtcaattgaccgtcaacatcgacaatatcgcgatcagcgattcggacagtaattctgatatcaacatcggtatggtcga
tggcgaagaatttttgttcagcgtaaatacgccagtggcgctgtataccaatattatatttgatttgaagcaaaacgatgatgtc
attcaaaccggcatcgtgctaacgccgaccgaaaacaacggcggttcgtttgaaaacggcattacctccgatgccgacaa
ccacatcatcgccggtcgtcctgaattgctgcacggcgcctacatcgacggcggcgggggctacaacacgttggaagtcg
acatgaaaggcttctttgcg

Slayer_{Nterm}-L1lip-ssp DnaB intein fusion, sequence
(the following 4 domains are linked to constitute the complete recombinant protein, however, the individual domains are separated from each other, below, so that the sequence of each domain can be identified) (SEQ ID NO:6):
*upstream sequence, non-coding* 
tttaacattctaaatgggcgcaagtccgtataaatttaccacgaatcgggtttaatcgcagagcggcgaggcatcgttttacaa
cccgcccggcaaagaaagtgttgtgggggcgacgcctacgtcgcgataaatcgaagtcgtgtcgctaatcgacaaaaca
aacgtactcgaagcccctacggcacgaagcccggcagcgtagcggaattcgggaatggcctggctccgaactccccgg
 
attgcgccatgctccatccgggctgcgctgtttagactcgccgaaagcggtcggtagcgatttatcagcgagaaaccttgtt
aactttatgcgtatgggcgacaacccccgtccggcataagggcggcaaagaaagtgttgtgggagcgacgcctacgtcg
cgataaatcgaagtcgtgtcgctaatcgacaaaacaaacggactcgaagcccctacggcacgaagcccggcagcgtagc
ggaattcgggaatggcctggctccgaactccccggattgcgccatgctccatccgggctacgttgtttagactcgccgaaa
gcggtcgctagcgatttatcagcgagaatctttgttagctttatgcgtatgggcgacaacccccgtccggcataagggcaac
aaaaagttcagctatggaatcgatatatcgataaataaacaatgaaaaaacattttaattaaatcaaatatataaacttaaatta
cactaaaatacttcaatcatagtaatagaaagccaaattttaagcattatttcacccaaaataagggcggtccctagaaaaatt
attaaaaactctctatactcaagcaccgtaagctatcaactcagtccagctctttacttagaaaggctgatcaaggtatagtgc
atacaaaattcagtgcgtatcaaaacgtgtctagagttctttctaacaaaaagcgaaaacctcaattggagatttaac
*L1 lipase* 
atggcctcgccgcgtgcgaacgatgcgccgattgtgctgttacatggttttacgggctggggccgggaagaaatgctgggt
ttcaaatactggggcggcgtccgcggcgatatcgaacaatggttgaatgataatggctatcgcacctataccttggccgtcg
gcccgttgtcgagcaattgggatcgcgcgtgcgaagcgtatgcccaattggtcggcggcaccgtcgattatggtgccgcg
catgccgcgaatgatggccatgcccgctttggccgcacctatccgggcttgttgccggaattgaaacgcggcggccgtgtc
catatcattgcccatagccaaggcggccaaacggcccgtatgttggtctcgttgttggaaaatggcagccaagaagaacgc
gaatatgccaaagaacataatgtctcgttgagcccgttgtttgaaggcggccatcgcttcgtcttgtcggtcaccaccatcgc
caccccgcatgatggcaccaccttggtcaatatggtcgattttaccgatcgctttttcgatttgcaaaaagccgtcttggaagc
cgccgcagtcgcgtcgaatgccccgtacaccagcgaaatttatgatttcaaattggatcaatggggcttgcgtcgcgaacc
gggcgaatcgtttgatcattatttcgaacgcttgaaacgctcgccggtctggaccagcacggatacggcccgctatgatttg
agcgtcccgggcgccgaaaccttgaatcgctgggtcaaagcgtcgccgaatacctattatttgtcgttcagcaccgaacgc
acctatcgtggcgccttgaccggcaattattatccggaattgggcatgaatgcgttttcggccatcgtctgcgcgccgttcttg
ggcagctatcgcaatgcggccttgggcattgattcgcattggttgggcaatgatggcatcgtcaataccatttcgatgaatgg
cccgaaacgcggcagcaatgatcgcatcgtcccgtatgatggcaccttgaagaaaggcgtctggaatgatatgggcacct
ataaagtcgatcatttggaagtcattggcgtcgatccgaatccgtcgttcaacattcgtgcgttttatctgcgtttagcggaaca
actggcgtccctgcgtccg
*Ssp DnaB intein* 
tgtagagcaatggccatcagcggcgattcgttgatctcgttggcctcgaccggcaaacgcgtcagcatcaaagatttgttgg
atgaaaaagatttcgaaatctgggccattaatgaacaaaccatgaaattggaaagcgcgaaagtctcgcgcgtcttctgcac
cggcaaaaaattggtctatatcttgaaaacccgcttgggccgcaccattaaagccaccgcgaatcatcgctttttgaccatcg
atggctggaaacgcttggatgaattgagcttgaaagaacatattgccttgccgcgcaaattggaatcgtcgtcgttgcaattgt
cgccggaaatcgaaaaattgagccaatcggatatttattgggatagcatcgtctcgattaccgaaaccggcgtcgaagaagt
ctttgatttgaccgtcccgggcccgcataatttcgtcgcgaatgatattattgtccataat
*S layer protein (in frame with L1 lip)*

Gcaacactctcagtggatatcgctcaatcctatatggagaccttacggtcctatgggcttgagtttaacataagacaaaccaa
caacctgaccaatcggataattaaccgcttggaaaatcgcggccacacacctgagcaagtagcagactggcttatgagca
gggttgcggttaaacagcaattgagaaaactggttaaacaaggcgaattggccgagtttgatctggatggcaatggccgcc
tcaatcgatctgaattgctaaatgcaatgtctgctctgtccgagacagcggtcgaagaagcgccggtcgaagatcccacga
ctcccaagcctccggccgatcctagcatcacgaccttaacgcttactgagattccgactcagcgtacggctacgttaaaatg
gaacaatgtcgatgccgatttggccatcgatttcatgcaagacgtactgaaactagatctaaatcgactaggctggatggaa
gacggtcaattgaccgtcaacatcgacaatatcgcgatcagcgattcggacagtaattctgatatcaacatcggtatggtcga
tggcgaagaatttttgttcagcgtaaatacgccagtggcgctgtataccaatattatatttgatttgaagcaaaacgatgatgtc
attcaaaccggcatcgtgctaacgccgaccgaaaacaacggcggttcgtttgaaaacggcattacctccgatgccgacaa
ccacatcatcgccggtcgtcctgaattgctgcacggcgcctacatcgacggcggcgggggctacaacacgttggaagtcg
acatgaaaggcttctttgcg

Slayer_{Nterm}-L1lip-Mxe GyrA intein fusion, sequence
(the following 4 domains are linked to constitute the complete recombinant protein, however, the individual domains are separated from each other, below, so that the sequence of each domain can be identified) (SEQ ID NO:7):
*upstream sequence, non-coding* 
tttaacattctaaatgggcgcaagtccgtataaatttaccacgaatcgggtttaatcgcagagcggcgaggcatcgttttacaa
cccgcccggcaaagaaagtgttgtgggggcgacgcctacgtcgcgataaatcgaagtcgtgtcgctaatcgacaaaaca
aacgtactcgaagcccctacggcacgaagcccggcagcgtagcggaattcgggaatggcctggctccgaactccccgg
attgcgccatgctccatccgggctgcgctgtttagactcgccgaaagcggtcggtagcgatttatcagcgagaaaccttgtt
aactttatgcgtatgggcgacaaccccgtccggcataagggcggcaaagaaagtgttgtgggagcgacgcctacgtcg
cgataaatcgaagtcgtgtcgctaatcgacaaaacaaacggactcgaagcccctacggcacgaagcccggcagcgtagc
ggaattcgggaatggcctggctccgaactccccggattgcgccatgctccatccgggctacgttgtttagactcgccgaaa
gcggtcgctagcgatttatcagcgagaatctttgttagctttatgcgtatgggcgacaaccccgtccggcataagggcaac
aaaaagttcagctatggaatcgatatatcgataaataaacaatgaaaaaacattttaattaaatcaaatatataaaacttaaatta
cactaaaatacttcaatcatagtaatagaaagccaaattttaagcattatttcacccaaaataagggcggtccctagaaaaatt
attaaaaactctctatactcaagcaccgtaagctatcaactcagtccagctctttacttagaaaggctgatcaaggtatagtgc
atacaaaattcagtgcgtatcaaaacgtgtctagagttctttctaacaaaaagcgaaaacctcaattggagatttaac
*L1 lipase* 
atggcctcgccgcgtgcgaacgatgcgccgattgtgctgttacatggttttacgggctggggccgggaagaaatgctgggt
ttcaaatactggggcggcgtccgcggcgatatcgaacaatggttgaatgataatggctatcgcacctataccttggccgtcg
gcccgttgtcgagcaattgggatcgcgcgtgcgaagcgtatgcccaattggtcggcggcaccgtcgattatggtgccgcg
catgccgcgaatgatggccatgcccgctttggccgcacctatccgggcttgttgccggaattgaaacgcggcggccgtgtc
 
catatcattgcccatagccaaggcggccaaacggcccgtatgttggtctcgttgttggaaaatggcagccaagaagaacgc
gaatatgccaaagaacataatgtctcgttgagcccgttgtttgaaggcggccatcgcttcgtcttgtcggtcaccaccatcgc
caccccgcatgatggcaccaccttggtcaatatggtcgattttaccgatcgctttttcgatttgcaaaaagccgtcttggaagc
cgccgcagtcgcgtcgaatgccccgtacaccagcgaaatttatgatttcaaattggatcaatggggcttgcgtcgcgaacc
gggcgaatcgtttgatcattatttcgaacgcttgaaacgctcgccggtctggaccagcacggatacggcccgctatgatttg
agcgtcccgggcgccgaaaccttgaatcgctgggtcaaagcgtcgccgaatacctattatttgtcgttcagcaccgaacgc
acctatcgtggcgccttgaccggcaattattatccggaattgggcatgaatgcgttttcggccatcgtctgcgcgccgttcttg
ggcagctatcgcaatgcggccttgggcattgattcgcattggttgggcaatgatggcatcgtcaataccatttcgatgaatgg
cccgaaacgcggcagcaatgatcgcatcgtcccgtatgatggcaccttgaagaaaggcgtctggaatgatatgggcacct
ataaagtcgatcatttggaagtcattggcgtcgatccgaatccgtcgttcaacattcgtgcgttttatctgcgtttagcggaaca
actggcgtccctgcgtccg
*Mxe GyrA intein* 
gcaatgcgcatgtgcatcaccggcgatgcgttggtcgccttgccggaaggcgaatcggtccgtattgccgatattgtcccg
ggcgcccgtccgaatagcgataatgcgattgatttgaaagtcttggatcgtcatggcaatccggtcttggccgatcgcttgtt
ccattcgggcgaacatccggtctataccgtccgtacggtcgaaggtttgcgtgtcacgggcaccgcgaatcatccgttgttg
tgcttggtcgatgtcgccggcgtcccgaccttgttgtggaaattgatcgatgaaatcaaaccgggcgattatgcggtcatcca
acgctcggccttttcggtcgattgcgccggttttgcccgtggcaaaccggaatttgccccgaccacctatacggtcggcgtc
ccgggtttggtccgcttcttggaagcccatcatcgcgatccggatgcccaagcgatcgccgatgaattgaccgatggccgc
ttttattatgcgaaagtcgcctcggtcacggatgcgggcgtccaaccggtctatagcttgcgcgtcgataccgcggatcatg
cctttattaccaatggcttcgtcagccatgcc
*S layer protein (in frame with L1 lip)*

gcaacactctcagtggatatcgctcaatcctatatggagaccttacggtcctatgggcttgagtttaacataagacaaaccaa
caacctgaccaatcggataattaaccgcttggaaaatcgcggccacacacctgagcaagtagcagactggcttatgagca
gggttgcggttaaacagcaattgagaaaactggttaaacaaggcgaattggccgagtttgatctggatggcaatggccgcc
tcaatcgatctgaattgctaaatgcaatgtctgctctgtccgagacagcggtcgaagaagcgccggtcgaagatcccacga
ctcccaagcctccggccgatcctagcatcacgaccttaacgcttactgagattccgactcagcgtacggctacgttaaaatg
gaacaatgtcgatgccgatttggccatcgatttcatgcaagacgtactgaaactagatctaaatcgactaggctggatggaa
gacggtcaattgaccgtcaacatcgacaatatcgcgatcagcgattcggacagtaattctgatatcaacatcggtatggtcga
tggcgaagaatttttgttcagcgtaaatacgccagtggcgctgtataccaatattatatttgatttgaagcaaaacgatgatgtc
attcaaaccggcatcgtgctaacgccgaccgaaaacaacggcggttcgtttgaaaacggcattacctccgatgccgacaa
ccacatcatcgccggtcgtcctgaattgctgcacggcgcctacatcgacggcggcgggggctacaacacgttggaagtcg
acatgaaaggcttctttgcg

### REFERENCES EXAMPLE 2

1. Anthony, C. 1982. The biochemistry of methylotrophs.London ; New York : Academic Press 431p.
2. Demidenko A, et al. 2017. Fatty Acid Biosynthesis Pathways in Methylomicrobium buryatense 5G(B1). Front Microbiol. 17:2167.
3. Egelseer, E.M., et al. 2009. S-Layers, Microbial, Biotechnological Applications, in: Encyclopedia of Industrial Biotechnology, John Wiley & Sons, Inc.
4. Graf R, et al. 2008. The multifunctional role of ectoine as a natural cell protectant. Clin Dermatol. 26 :326-33.
5. Henard CA, et al. 2016. Bioconversion of methane to lactate by an obligate methanotrophic bacterium. Sci Rep. 2016 6:21585. doi: 10.1038/srep21585.
6. He Y-Z, et al. 2015. High production of ectoine from aspartate and glycerol by use of whole-cell biocatalysis in recombinant Escherichia coli. Microbial Cell Factories 14:55.
7. Jeffries P, Wilkinson JF. Electron Microscopy of the cell wall complex of Methylomonas albus. Arch Microbiol 1978; 119:227-29.
8. Kalyuzhnaya M.G., Puri A., & Lidstrom M.E. 2015. Metabolic engineering in methanotrophic bacteria. Metab Eng. 29, 142-52.
9. Kim HK, et al. 1998. Gene cloning and characterization of thermostable lipase from Bacillus stearothermophilus L1. Biosci Biotechnol Biochem. 62(1):66-71.
10. Kim MH, et al. 2000. Thermostable lipase of Bacillus Stearothermophilus: high-level production, purification, and calcium-dependent thermostability. Biosci Biotechnol Biochem. 64:280-6.
11. Khmelenina, V.N., et al. 1992. The synthesis of polysaccarides by Methylococcus capsulatus under various conditions of cultivation. Mikrobiologiia (Russian). 61, 404-410.
12. Khmelenina VN, Kalyuzhnaya MG, Sakharovsky VG et al. Osmoadaptation in halophilic and alkaliphilic methanotrophs, Arch Microbiol 1999;172:321-29.
13. Khmelenina VN, et al. Structural and functional features of methanotrophs from hypersaline and alkaline lakes. Microbiology (Russia) 2010;9:472-82.
14. Mustakhimov I. I., et al. 2010. Identification and characterization of EctR, a new transcriptional regulator of the ectoine biosynthesis genes in the halotolerant methanotroph Methylomicrobium alcaliphilum 20Z. J. Bacteriol. 192, 410-7.
15. Ojala, D.S., et al. Genetic systems for moderately halo(alkali)philic bacteria of the genus Methylomicrobium. Methods Enzymol. Methods Enzymol. 495, 99-118.
16. Pastor JM, et al, 2010. Ectoines in cell stress protection: uses and biotechnological production. Biotechnol. Adv. 28 782-801.
17. Puri, A.W., et al. 2015. Genetic tools for the industrially promising methanotroph Methylomicrobium buryatense. Appl Environ Microbiol. 81, 1775-81.
18. Reshetnikov AS, et al. 2011. Genes and enzymes of ectoine biosynthesis in halotolerant methanotrophs. Methods in Enzymol. 495:15-30.
19. Riis, V., et al. 2003. Highly sensitive determination of ectoine and other compatible solutes by anion-exchange chromatography and pulsed amperometric detection. Anal. Bioanal. Chem. 377:203-207.
20. Strong PJ, et al. 2016. A methanotroph-based biorefinery: Potential scenarios for generating multiple products from a single fermentation. Bioresour Technol. 215:314-23.
21. Trotsenko, Y. A., et al. Biotechnological potential of aerobic methylotrophic bacteria: a review of current state and future prospects. Appl. Biochem. Microbiol. 2005; 41, 433-441.
22. Vuilleumier S. , et al. 2012. Genome Sequence of the Haloalkaliphilic Methanotrophic Bacterium Methylomicrobium alcaliphilum 20Z. J Bacteriol. 194, 551-2.

## Claims

1. A method for making a chimeric polypeptide comprising the major S-laver polypeptide of Methylomicrobium alcaliphilum sp.20Z, a self-assembling fragment thereof, and a heterologous polypeptide or peptide, the method comprising recombinantly engineering Methylomicrobium alcaliphilum sp. 20Z to recombinantly express said chimeric polypeptide and deliver it outside of the cell where it exists in the form of an assembled monomolecular layer.

2. A genetically engineered Methylomicrobium alcaliphilum sp. 20Z comprising an expression cassette which recombinantly expresses the chimeric protein of claim 1 and delivers it outside of the cell where it exists in the form of an assembled monomolecular layer.

3. An isolated assembled monomolecular layer comprising the chimeric protein of claim 1.

## Patentansprüche

1. Verfahren zur Herstellung eines chimären Polypeptids, das das Haupt-S-Schicht-Polypeptid von Alkaliphilum Dimetilomicrobium sp.20Z oder ein selbstassemblierendes Fragment davon und ein heterologes Polypeptid oder Peptid umfasst, wobei das Verfahren die rekombinante Technik von Alkaliphilum Dimetilomicrobium sp.20Z umfasst, um ein chimäres Polypeptid rekombinant zu exprimieren und es aus der Zelle hinaus zu schicken, wo es in Form einer zusammengesetzten monomolekularen Schicht vorliegt.

2. Genetisch verändertes Alkaliphilum Dimetilomicrobium sp.20Z, umfassend eine Expressionskassette, die das chimäre Protein nach Anspruch 1 rekombinant exprimiert und es aus der Zelle hinausschickt, wo es in Form einer monomolekularen Schichtanordnung vorliegt.

3. Zusammengebaute und isolierte monomolekulare Schicht, umfassend das chimäre Protein nach Anspruch 1.

## Revendications

1. Procédé de fabrication d'un polypeptide chimère comprenant le polypeptide principal de couche S de Alkaliphilum Dimetilomicrobium sp.20Z, ou un fragment auto-assemblant de celui-ci, et un polypeptide ou peptide hétérologue, le procédé comprenant l'ingénierie recombinante d'Alkaliphilum Dimetilomicrobium sp.20Z pour exprimer de manière recombinante un polypeptide chimère et pour l'envoyer à l'extérieur de la cellule, ou il existe en forme d'une couche monomoleculaire assemblé.

2. Alkaliphilum Dimetilomicrobium sp.20Z génétiquement modifié comprenant une cassette d'expression exprimant de manière recombinante la protéine chimère de la revendication 1, et l'envoie à l'extérieur de la cellule ou celle-ci est présente en forme d'une couche mono-moléculaire assemblée.

3. Couche mono-moléculaire assemblée et isolée comprenant la protéine chimère de la revendication 1.
